# EUROPEAN PATENT SPECIFICATION

(11) **EP 4 142 878 B1**
(45) Date of publication and mention of the grant of the patent: **26.06.2024**
(21) Application number: 21724470.6
(22) Date of filing: 20.04.2021
(51) Int. Cl.: A61P 3/10, A61P 13/12, A61P 25/00, A61P 25/16, A61K 31/40, C07D 401/06

(54) **PYRIDINE INHIBITORS OF GLUCOSYLCERAMIDE SYNTHASE AND THERAPEUTIC METHODS USING THE SAME**
PYRIDINHEMMER DER GLUCOSYLCERAMIDSYNTHASE UND THERAPEUTISCHE VERFAHREN DAMIT
INHIBITEURS PYRIDINE DE LA GLUCOSYLCÉRAMIDE SYNTHASE ET PROCÉDÉS THÉRAPEUTIQUES LES UTILISANT

(30) Priority: 28.04.2020 US 202063016683 P
(43) Date of publication of application: 08.03.2023
(73) Proprietor: The Regents of The University of Michigan, Ann Arbor, MI 48109-2590 (US)
(72) Inventor: LARSEN, Scott D., South Lyon MI 48178 (US); SHAYMAN, James A., Ann Arbor, MI 48103 (US); WILSON, Michael William, Ann Arbor, MI 48105 (US); MOSS, Neil, Ridgefield, CT 06877 (US)
(74) Representative: Studio Torta S.p.A.
(86) International application number: PCT/US2021/028136
(87) International publication number: WO 2021/221953

(56) References cited:
- WO-A1-2016/145153
- WO-A1-2021/081141
- WO-A2-2016/126572
- S. D. LARSEN ET AL: "Property-based design of a glucosylceramide synthase inhibitor that reduces glucosylceramide in the brain", THE JOURNAL OF LIPID RESEARCH, vol. 53, no. 2, 1 February 2012 (2012-02-01), pages 282-291, XP055159312, ISSN: 0022-2275, DOI: 10.1194/jlr.M021261

## Description

### FIELD OF THE INVENTION

The present invention relates to pyridine inhibitors of glucosylceramide synthase (GCS) and to uses in treating conditions and diseases wherein inhibition of GCS provides a benefit.

### BACKGROUND OF THE INVENTION

Lysosomal storage diseases (LSDs), such as Gaucher disease and Fabry disease, occur when glycolipids accumulate in lysosomes due to defect in their catabolism. Two general strategies exist for the treatment of lysosomal storage diseases. The first strategy includes replacement or restoration of the defective or absent catabolizing enzyme (e.g., the infusion of recombinant enzyme, chaperone therapy, bone marrow transplantation, or gene therapy) (1). Enzyme replacement therapy is clinically approved for lysosomal storage diseases with peripheral manifestations, but is limited by the inability of the infused recombinant enzyme to distribute into the CNS, and by the frequent development of auto-antibodies to the protein in patients carrying null mutations.

The second strategy involves synthesis inhibition therapy focused on identifying small molecule inhibitors of GCS (2). Various classes of GCS inhibitors have been described, including imino sugars and analogues of D-*threo*-1-phenyl-2-decanoylamino-3-morpholino-propanol (PDMP) (3). The imino sugar N-butyldeoxynojirimycin (NBDNJ) is limited by its micromolar level inhibitory activity and limited specificity against the synthase. The limited specificity is associated with a high level of undesired effects resulting from secondary sites of action unrelated to glycolipid synthesis inhibition. These effects most notably include diarrhea, weight loss, and tremor, which limits the approved use of NBDNJ in the United States (4). One advantage of NBDNJ over PDMP-based homologs reported to date is its ability to distribute into the CNS. However, a recent study raised questions with respect to the ability of NBDNJ to lower CNS glycolipid levels (K.M. Ashe et al., Plos One 6:e21758 (2011)).

A number of GCS inhibitors have been disclosed, for example, in U.S. Patent Nos. 5,302,609; 5,472,969; 5,525,616; 5,916,911; 5,945,442; 5,952,370; 6,030,995; 6,051,598; 6,255,336; 6,569,889; 6,610,703; 6,660,794; 6,855,830; 6,916,802; 7,253,185; 7,196,205; 7,615,573; 7,994,198; 10,189,784; 10,202,340; and U.S. Patent Publications 2008/0234324; 2016/0280643; 2018/0044302; 2018/0093981; and 2019/0031652. Additional GCS inhibitors and treatments are disclosed in WO 2003/035626; WO 2008/150486; WO 2009/117150; WO 2010/014554; WO 2010/091104; WO 2010/0091164; WO 2012/129084; WO 2015/042397; WO 2015/065937; WO 2017/204319; and EP 3 318 277.

A compound structurally related to PDMP is N-((1R,2R)-1-(2,3-dihydrobenzo[b][1,4]dioxin-6-yl)-1-hydroxy-3-(pyrrolidin-1-yl)propan-2-yl)octanamide, also known as Genz-112638 and eliglustat tartrate (5). Phase 2 clinical trials using this drug for type 1 Gaucher disease demonstrated an efficacy equal to or greater than that for recombinant β-glucocerebrosidase, as evidenced by reversal of spleen and liver enlargement, correction of anemia, and improvements in thrombocytopenia and bone density (6). Phase 3 trials with eliglustat tartrate have been published, and eliglustat tartrate has been approved worldwide for Gaucher disease type 1 (7).

GSC inhibition also is expected to treat six other lysosomal storage diseases with CNS involvement, including early and late onset Tay-Sachs disease, Sandhoff disease, GM1 gangliosidosis, and types 2 and 3 Gaucher disease. For example, an experimental model of genetic epistasis demonstrated markedly improved survival in a mouse model of Sandhoff disease that also lack GM2 synthase (8). However, drug distribution studies indicate that eliglustat tartrate is not transported across the blood brain barrier (BBB) (5). A possible basis for the poor brain distribution of eliglustat tartrate may be that the drug is a substrate for the p-glycoprotein (MDR1) transporter, resulting in efflux of the drug (Larsen et al, J Lipid Res 2012, 53, 282-91). The absence of brain penetrance is clinically advantageous for diseases that have no neurological involvement. These include the two most prevalent lysosomal storage diseases, Gaucher disease type 1 and Fabry disease. Miglustat, an oral agent approved for Gaucher disease type 1 but restricted to only those patients who are intolerant of enzyme replacement therapies, has demonstrable brain penetrance but is plagued by several toxicities due to its off target activities, including neurologic ones such as tremors. These toxicities have not been observed in patients on eliglustat (Orphanet et al, J Rare Dis 2019, 14, 128).

Compounds that inhibit GCS have the potential to treat conditions associated with glycolipid accumulation. However, many GCS inhibitors are limited by poor CNS penetration and/or low activity. An important advance in the art would be the discovery of GCS inhibitors, and particularly GCS inhibitors capable of crossing the BBB, that are useful in the treatment of diseases wherein GCS inhibition provides a benefit, such as type II or III Gaucher disease, Tay-Sachs disease, Sandhoff disease, diabetes, lupus, and other diseases and conditions associated with glycolipid accumulation in lysosomes. Accordingly, a need still exists in the art for efficacious compounds and compositions useful in the treatment of such diseases, alone or in conjunction with other therapies used to treat these diseases and conditions. The present invention is directed to meeting this need.

### SUMMARY OF THE INVENTION

The present invention is directed to inhibitors of GCSto compositions comprising the inhibitors, and to uses of the inhibitors in a therapeutic treatment of conditions and diseases wherein inhibition of GCS provides a benefit. The present compounds are potent inhibitors of GCS, and in some embodiments are capable of crossing the BBB and in some embodiments are poorly permeable through the BBB. Any references in the description to methods of treatment refer to the compounds, pharmaceutical compositions and medicaments of the present invention for use in a method for treatment of the human (or animal) body by therapy (or for diagnosis).

The invention is set out in the appended set of claims.

More particularly, the present invention is directed to compounds having a structural formula (I): 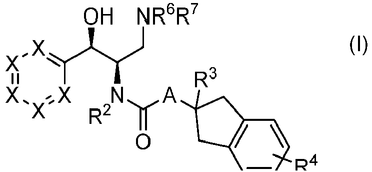
wherein each X independently is CH, CR¹, or N, and with the proviso that one and only one X must be N;
A is CH₂ or O;
R¹ is H, halogen, or OR⁵;
R² is H, C₁-C₅ alkyl, or C₃-C₆ cycloalkyl;
R³ is H or CH₃;
R⁴ is H or F;
R⁵ is C₁-C₄ alkyl or C₃-C₆ cycloalkyl, optionally substituted with one or more F; and
R⁶ and R⁷ independently, are H or C₁-C₃ alkyl or R⁶ and R⁷ are taken together with the nitrogen atom to which they are attached to form a C₄-C₈ heterocycloalkyl or heterobicycloalkyl ring, optionally substituted with F or CH₃;
or a pharmaceutically acceptable salt thereof.

In one embodiment, the present invention provides a use in treating a condition or disease of interest by administering a therapeutically effective amount of a compound of structural formula (I) to an individual in need thereof. The disease or condition, for example, Gaucher disease, Fabry disease, Sandhoff disease, Tay-Sachs disease, and Parkinson's disease, is treatable by inhibition of GCS.

In yet another embodiment, the present invention provides a use in treating a subject having type 2 diabetes comprising administering to the subject a therapeutically effective amount of a compound of structural formula (I).

The use in treating a subject having renal hypertrophy or hyperplasia, hyperplasia associated with diabetic nephropathy, or autosomal dominate polycystic kidney disease (ADPKD) or a viral illness also is included in the invention. The use comprises administering to the subject a therapeutically effective amount of a compound of structural formula (I).

A use in decreasing plasma TNF-α in a subject in need thereof also is included in the present invention. Indications treatable by the compounds of structural formula (I) also include, but are not limited to, multiple myeloma (for depletion of glucosylsphinogosine) and inhibition of viral and bacterial illnesses, the latter including hemolytic uremic syndrome where depletion of the receptor for shiga toxin, i.e., globotriaosylceramide, may be depleted from vascular endothelial cells. The use comprises administering to the subject a therapeutically effective amount of a compound of structural formula (I).

A use in lowering blood glucose levels in a subject in need thereof also is included in the present invention. The use comprises administering to the subject a therapeutically effective amount of a compound of structural formula (I).

The use in decreasing glycated hemoglobin levels in a subject in need thereof also is included in the present invention. The use comprises administering to the subject a therapeutically effective amount of a compound of structural formula (I).

A use in inhibiting glucosylceramide synthase or lowering glycosphingolipid concentrations in a subject in need thereof also is included in the present invention. The use comprises administering to the subject a therapeutically effective amount of a compound of structural formula (I).

The present invention also is directed to of the use in treating a glomerular disease selected from the group consisting of mesangial proliferative glomerulonephritis, collapsing glomerulopathy, proliferative lupus nephritis, crescentic glomerulonephritis, and membranous nephropathy in a subject, comprising administering to the subject a therapeutically effective amount of a compound of structural formula (I).

In another embodiment, the invention is directed to the use in treating lupus in a subject comprising administering to the subject a therapeutically effective amount of a compound of structural formula (I).

In yet another embodiment, in the treatment of the above disclosed diseases, a compound of structural formula (I) can be administered as the sole therapeutic agent or in combination with a second therapeutic agent known to treat the disease of interest.

Another embodiment of the present invention is to provide a composition comprising (a) a GCS inhibitor of structural formula (I) and (b) an excipient and/or pharmaceutically acceptable carrier useful in treating diseases or conditions wherein inhibition of GCS provides a benefit.

Another embodiment of the present invention is to utilize a composition comprising a compound of structural formula (I) and a second therapeutically active agent in of the use in treating an individual for a disease or condition wherein inhibition of GCS provides a benefit.

In a further embodiment, the invention provides for use of a composition comprising a GCS inhibitor of structural formula (I) and an optional second therapeutic agent for the manufacture of a medicament for treating a disease or condition of interest, e.g., Gaucher disease or Fabry disease.

Also disclosed is a kit for human pharmaceutical use comprising (a) a container, (b1) a packaged composition comprising a GCS inhibitor of structural formula (I), and, optionally, (b2) a packaged composition comprising a second therapeutic agent useful in the treatment of a disease or condition of interest, and (c) a package insert containing directions for use of the composition or compositions, administered simultaneously or sequentially, in the treatment of the disease or condition.

The GCS inhibitor of structural formula (I) and the second therapeutic agent can be administered together as a single-unit dose or separately as multi-unit doses, wherein the GCS inhibitor of structural formula (I) is administered before the second therapeutic agent or vice versa. It is envisioned that one or more dose of a GCS inhibitor of structural formula (I) and/or one or more dose of a second therapeutic agent can be administered.

In one embodiment, a GCS inhibitor of structural formula (I) and a second therapeutic agent are administered simultaneously. In related embodiments, a GCS inhibitor of structural formula (I) and second therapeutic agent are administered from a single composition or from separate compositions. In a further embodiment, the GCS inhibitor of structural formula (I) and second therapeutic agent are administered sequentially. A GCS inhibitor of structural formula (I), as used in the present invention, can be administered in an amount of about 0.005 to about 500 milligrams per dose, about 0.05 to about 250 milligrams per dose, or about 0.5 to about 100 milligrams per dose.

Compounds of the invention inhibit GCS and are useful research tools for *in vitro* study of GCS and its role in biological process.

These and other novel aspects of the present invention will become apparent from the following detailed description of the present embodiments.

### DETAILED DESCRIPTION OF THE PREFERRED EMBODIMENTS

The term "GCS" as used herein means glucosylceramide synthase.

The term "a disease or condition wherein inhibition of GCS provides a benefit" pertains to a condition in which GCS, and/or an action of GCS, is important or necessary, e.g., for the onset, progress, expression of that disease or condition, or a disease or a condition which is known to be treated by a GCS inhibitor (such as eliglustat tartrate). An example of such a condition includes, but is not limited to, Gaucher disease and Fabry disease. One of ordinary skill in the art is readily able to determine whether a compound treats a disease or condition mediated by GCS, for example, by assays which conveniently can be used to assess the activity of particular compounds.

The term "second therapeutic agent" refers to a therapeutic agent different from a GCS inhibitor of structural formula (I) and that is known to treat the disease or condition of interest. In particular, a GCS inhibitor of structural formula (I) can be used to act in synergy with second therapeutic agents that increase the activity of beta-glucocerebrosidase. For example when Gaucher disease is the disease or condition of interest, the second therapeutic agent can be a known for the treatment of type (I) Gaucher disease or Fabry disease, like isofagamine, enzyme replacement therapy, or gene therapy for example.

The term "disease" or "condition" denotes disturbances and/or anomalies that as a rule are regarded as being pathological conditions or functions, and that can manifest themselves in the form of particular signs, symptoms, and/or malfunctions. As demonstrated below, a compound of structural formula (I) is an inhibitor of GCS and can be used in treating diseases and conditions wherein inhibition of GCS provides a benefit.

As used herein, the terms "treat," "treating," "treatment," and the like refer to eliminating, reducing, or ameliorating a disease or condition, and/or symptoms associated therewith. Although not precluded, treating a disease or condition does not require that the disease, condition, or symptoms associated therewith be completely eliminated. As used herein, the terms "treat," "treating," "treatment," and the like may include "prophylactic treatment," which refers to reducing the probability of redeveloping a disease or condition, or of a recurrence of a previously-controlled disease or condition, in a subject who does not have, but is at risk of or is susceptible to, redeveloping a disease or condition or a recurrence of the disease or condition. The term "treat" and synonyms contemplate administering a therapeutically effective amount of a compound of the invention to an individual in need of such treatment.

Within the meaning of the invention, "treatment" also includes relapse prophylaxis or phase prophylaxis, as well as the treatment of acute or chronic signs, symptoms and/or malfunctions. The treatment can be orientated symptomatically, for example, to suppress symptoms. It can be effected over a short period, be oriented over a medium term, or can be a long-term treatment, for example within the context of a maintenance therapy.

The term "therapeutically effective amount" or "effective dose" as used herein refers to an amount of the active agent(s) that is(are) sufficient, when used according to the invention, to efficaciously deliver the active agent(s) for the treatment of condition or disease of interest to an individual in need thereof. In the case of a lysosomal storage disorder, the therapeutically effective amount of the agent may reduce (i.e., retard to some extent and preferably stop) unwanted glycolipid accumulation and/or relieve, to some extent, one or more of the symptoms associated with the disorder.

The term "container" means any receptacle and closure therefor suitable for storing, shipping, dispensing, and/or handling a pharmaceutical product.

The term "insert" means information accompanying a pharmaceutical product that provides a description of how to administer the product, along with the safety and efficacy data required to allow the physician, pharmacist, and patient to make an informed decision regarding use of the product. The package insert generally is regarded as the "label" for a pharmaceutical product.

"Concurrent administration," "administered in combination," "simultaneous administration," and similar phrases mean that two or more agents are administered concurrently to the subject being treated. By "concurrently," it is meant that each agent is administered either simultaneously or sequentially in any order at different points in time. However, if not administered simultaneously, it is meant that they are administered to an individual in a sequence and sufficiently close in time so as to provide the desired therapeutic effect and can act in concert. For example, a GCS inhibitor of structural formula (I) can be administered at the same time or sequentially in any order at different points in time as a second therapeutic agent. A present GCS inhibitor and the second therapeutic agent can be administered separately, in any appropriate form and by any suitable route. When a present GCS inhibitor and the second therapeutic agent are not administered concurrently, it is understood that they can be administered in any order to a subject in need thereof.

For example, a present GCS inhibitor can be administered prior to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapeutic agent treatment modality (e.g., radiotherapy), to an individual in need thereof. In various embodiments, a GCS inhibitor of structural formula (I) and the second therapeutic agent are administered 1 minute apart, 10 minutes apart, 30 minutes apart, less than 1 hour apart, 1 hour apart, 1 hour to 2 hours apart, 2 hours to 3 hours apart, 3 hours to 4 hours apart, 4 hours to 5 hours apart, 5 hours to 6 hours apart, 6 hours to 7 hours apart, 7 hours to 8 hours apart, 8 hours to 9 hours apart, 9 hours to 10 hours apart, 10 hours to 11 hours apart, 11 hours to 12 hours apart, no more than 24 hours apart or no more than 48 hours apart. In one embodiment, the components of the combination therapies are administered at 1 minute to 24 hours apart.

The use of the terms "a", "an", "the", and similar referents in the context of describing the invention (especially in the context of the claims) are to be construed to cover both the singular and the plural, unless otherwise indicated. Recitation of ranges of values herein merely are intended to serve as a shorthand method of referring individually to each separate value falling within the range, unless otherwise indicated herein, and each separate value is incorporated into the specification as if it were individually recited herein. The use of any and all examples, or exemplary language (e.g., "such as") provided herein, is intended to better illustrate the invention and is not a limitation on the scope of the invention unless otherwise claimed. No language in the specification should be construed as indicating any non-claimed element as essential to the practice of the invention.

Compounds that inhibit glycolipid synthesis are known. As such, these compounds can be used for treating diabetes, poylcystic kidney disease, Parkinson's disease, susceptible viral diseases and lysosomal storage diseases, such as Tay-Sachs disease, Sandhoff disease, Gaucher disease, and Fabry disease. However, to date, these compounds have been limited by low activity, poor CNS penetration, or both.

For example, glycolipid synthesis inhibition is the basis for the treatment of type 1 Gaucher disease by the glucosylceramide (GCS) inhibitor eliglustat tartrate. However, the use of eliglustat for the treatment of glycosphingolipid storage diseases with CNS manifestations is limited by the lack of brain penetration of this drug.

Phase 2 and phase 3 clinical data for eliglustat tartrate demonstrated a clinical response in type 1 Gaucher disease that is non-inferior to or superior to enzyme replacement therapy, as measured by reduction in spleen and liver volume, correction of anemia, and improvement in thrombocytopenia. The adverse effects observed with NBDNJ, including weight loss, diarrhea, and tremor, were not observed in this clinical trial, as well as in an extension study. These observations are consistent with the high specificity of eliglustat tartrate and its absence of CNS penetration, which may be advantageous for glycosphingolipidoses without CNS manifestations, including type 1 Gaucher and Fabry diseases. Some compounds of Formula 1 are poorly permeable through the BBB, giving them a similar advantage for the treatment of Gaucher and Fabry diseases. Conversely, the identification of compounds of structural formula (I) that cross the BBB is of therapeutic benefit for disorders such as GM2 gangliosidoses, Tay-Sachs, Sandhoff disease, and types 2 and 3 Gaucher disease, that exhibit CNS manifestations.

The GCS inhibitors of the present invention are novel and potent inhibitors of GCS, and therefore are useful in the treatment of diseases and conditions resulting from an unwanted accumulation of glycolipids, including Gaucher disease and type II diabetes. Also provided are uses in treating a subject having an unwanted accumulation of glycolipids comprising administering a therapeutically effective amount of a present compound to a subject in need of such treatment.

Also provided are uses in preventing the proliferation of unwanted glycolipid accumulation in a subject comprising the step of administering a therapeutically effective amount of a compound of structural formula (I) to a subject at risk of developing a condition characterized by unwanted glycolipid accumulation. In some embodiments, compounds of structural formula (I) are capable of crossing the BBB, therefore are useful in the treatment of lysosomal storage diseases that previously could not be treated by a stand-alone GCS inhibitor, for example, type II and type III Gaucher disease, Tay-Sachs and Sandhoff disease.

More particularly, the present invention is directed to compounds having a structural formula (I): 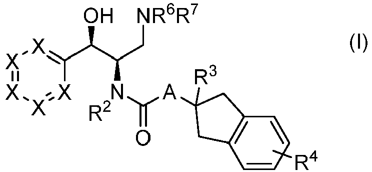
wherein each X independently is CH, CR¹, or N, and with the proviso that one and only one X must be N;
A is CH₂ or O;
R¹ is H, halogen, or OR⁵;
R² is H, C₁-C₅ alkyl, or C₃-C₆ cycloalkyl;
R³ is H or CH₃;
R⁴ is H or F;
R⁵ is C₁-C₄ alkyl or C₃-C₆ cycloalkyl, optionally substituted with one or more F; and
R⁶ and R⁷ independently, are H or C₁-C₃ alkyl or R⁶ and R⁷ are taken together with the nitrogen atom to which they are attached to form a C₄-C₈ heterocycloalkyl or heterobicycloalkyl ring, optionally substituted with F or CH₃;
or a pharmaceutically acceptable salt thereof.

The compounds of structural formula (I) are used in treating a disease or condition wherein inhibition of GCS provides a benefit, for example Gaucher disease, Fabry disease, Tay-Sachs disease, Sandhoff disease, diabetes, hypertrophy or hyperplasia associated with diabetic neuropathy, polycystic kidney disease, viral illness, lupus, increased plasma TNF-α, elevated glycated hemoglobin levels, and a glomerular disease. The use comprises administering a therapeutically effective amount of a compound of structural formula (I) to an individual in need thereof. The present uses also encompass administering a second therapeutic agent, including enzyme replacement therapy, to the individual in addition to the compound of structural formula (I). The second therapeutic agent is selected from drugs or biologics known as useful in treating the disease or condition afflicting the individual in need thereof. Such diseases and conditions include, but are not limited to, Parkinson disease, multiple myeloma, and ADPKD.

As used herein, the terms "C₁-C₅ alkyl" and "C₁-C₄ alkyl" refer to straight chained and branched saturated hydrocarbon groups, nonlimiting examples of which include methyl, ethyl, straight chain and branched propyl groups, straight chain and branched butyl groups, and straight chain and branched pentyl groups.

The terms "C₃-C₆ cycloalkyl", "C₄-C₈ heterocycloalkyl or heterobicycloalkyl" refer to saturated cycloalkyl and heterocycloalkyl groups containing 4 to 8 atoms, including for example cyclopropyl, cyclobutyl, cyclopentyl, and cyclohexyl, and corresponding rings containing a nitrogen atom.

The alkyl, cycloalkyl, and heterocycloalkyl groups can be substituted with one or more F.

In various embodiments, 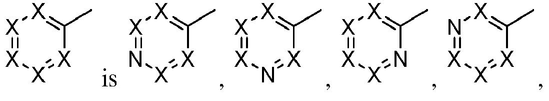 or 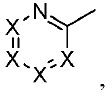 wherein X is CH or CR¹.

In one preferred embodiment, 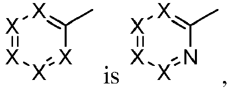 wherein X is CH or CR¹.

In various embodiments, when the pyridine ring system contains CR¹, then R¹ is - H, -OC(CH₃)₃, -OCH₃, -OCF₃, -OCH₂CF₃, or -Cl.

In preferred embodiments, R² is H or CH₃.

In another preferred embodiment, -NR⁶R⁷ is

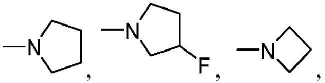

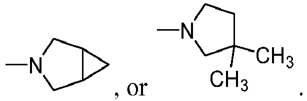

In various embodiments, 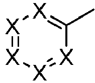 is

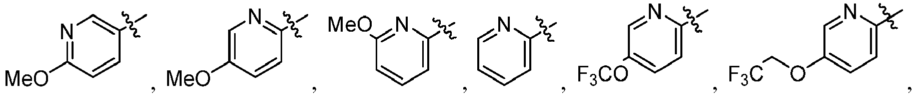

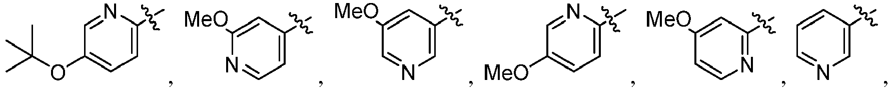

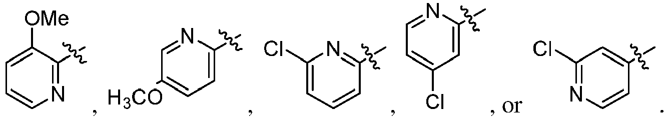

The present invention further includes all possible stereoisomers of the compounds of structural formula (I). The present invention includes both racemic compounds and optically active isomers. When a compound of structural formula (I) is desired as a single enantiomer, it can be obtained either by resolution of the final product or by stereospecific synthesis from either isomerically pure starting material or use of a chiral auxiliary reagent, for example, see Z. Ma et al., Tetrahedron: Asymmetry, 8(6), pages 883-888 (1997). Resolution of the final product, an intermediate, or a starting material can be achieved by any suitable method known in the art. Additionally, in situations where tautomers of the compounds of structural formula (I) are possible, the present invention is intended to include all tautomeric forms of the compounds.

Salts of the present compounds also are encompassed by the invention and can be used in the present invention. Pharmaceutically acceptable salts of the compounds of the invention often are preferred in the invention. As used herein, the term "pharmaceutically acceptable salts" refers to salts or zwitterionic forms of the compounds of structural formula (I). Salts of compounds of formula (I) can be prepared during the final isolation and purification of the compounds or separately by reacting the compound with an acid having a suitable cation. The pharmaceutically acceptable salts of compounds of structural formula (I) can be acid addition salts formed with pharmaceutically acceptable acids. Examples of acids which can be employed to form pharmaceutically acceptable salts include inorganic acids such as nitric, boric, hydrochloric, hydrobromic, sulfuric, and phosphoric, and organic acids such as oxalic, maleic, succinic, and citric. Nonlimiting examples of salts of compounds of the invention include, but are not limited to, the hydrochloride, hydrobromide, hydroiodide, sulfate, bisulfate, 2-hydroxyethansulfonate, phosphate, hydrogen phosphate, acetate, adipate, alginate, aspartate, benzoate, bisulfate, butyrate, camphorate, camphorsulfonate, digluconate, glycerolphosphate, hemisulfate, heptanoate, hexanoate, formate, succinate, fumarate, maleate, ascorbate, isethionate, salicylate, methanesulfonate, mesitylenesulfonate, naphthylenesulfonate, nicotinate, 2-naphthalenesulfonate, oxalate, pamoate, pectinate, persulfate, 3-phenylproprionate, picrate, pivalate, propionate, trichloroacetate, trifluoroacetate, phosphate, glutamate, glutarate, bicarbonate, paratoluenesulfonate, undecanoate, lactate, citrate, tartrate, gluconate, methanesulfonate, ethanedisulfonate, benzene sulphonate, and p-toluenesulfonate salts. In addition, available amino groups present in the compounds of the invention can be quaternized with methyl, ethyl, propyl, and butyl chlorides, bromides, and iodides; dimethyl, diethyl, dibutyl, and diamyl sulfates; decyl, lauryl, myristyl, and steryl chlorides, bromides, and iodides; and benzyl and phenethyl bromides. In light of the foregoing, any reference to compounds of the present invention appearing herein is intended to include compounds of structural formula (I) as well as pharmaceutically acceptable salts, hydrates, or solvates thereof.

Some specific embodiments of the present invention include, but are not limited to:

| **Example** | **Structure** |
|---|---|
| 1 | 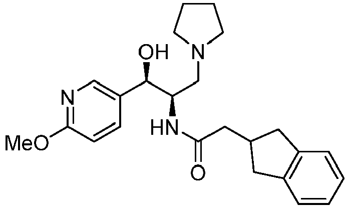 |
| 2 | 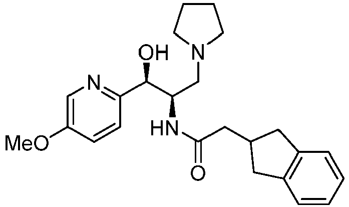 |
| 3 | 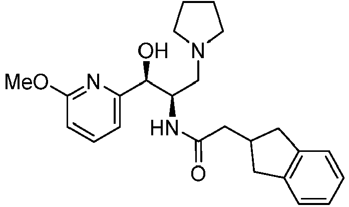 |
| 4 | 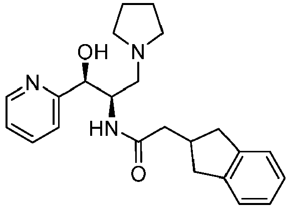 |
| 5 | 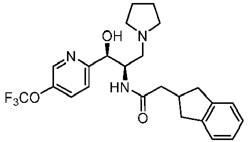 |
| 6 | 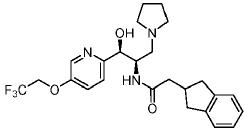 |
| 7 | 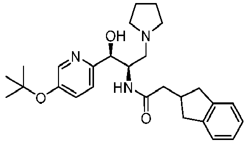 |
| 8 | 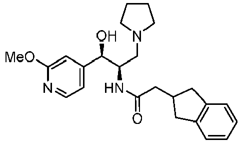 |
| 9 | 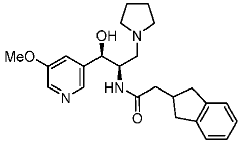 |
| 10 | 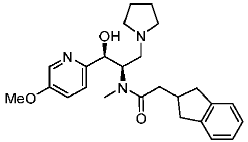 |
| 11 | 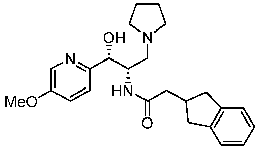 |
| 12 | 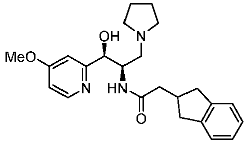 |
| 13 | 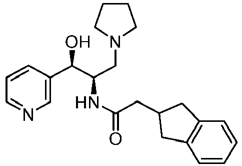 |
| 14 | 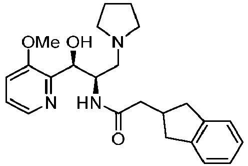 |
| 15 | 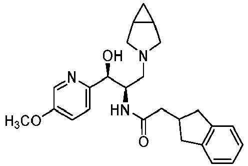 |
| 16 | |
| | 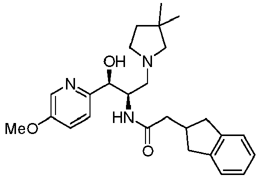 |
| 17 | 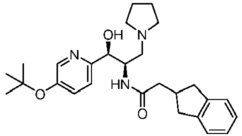 |
| 18 | 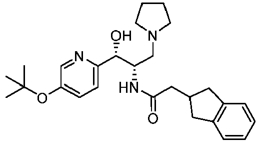 |
| 19 | 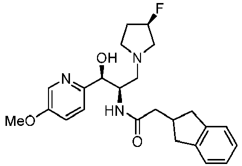 |
| 20 | 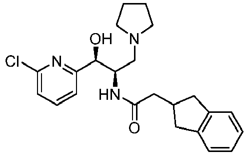 |
| 21 | 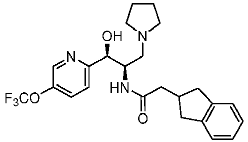 |
| 22 | 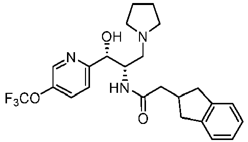 |
| 23 | 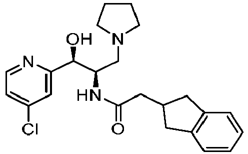 |
| 24 | 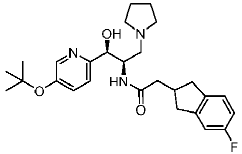 |
| 25 | 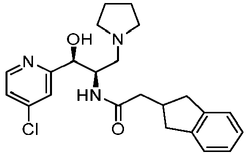 |
| 26 | 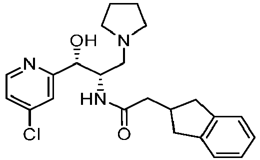 |
| 27 | 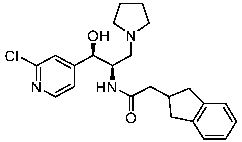 |

### SYNTHESIS OF COMPOUNDS

Compounds of the present invention were prepared as follows. The following synthetic schemes are representative of the reactions used to synthesize compounds of structural formula (I). Modifications and alternate schemes to prepare GCS inhibitors of the invention are readily within the capabilities of persons skilled in the art.

### Preparation and Spectroscopic Data of Compounds of Structural Formula (I)

Chemical names follow CAS or IUPAC nomenclature. Starting materials and reagents were purchased from Fisher, Sigma-Aldrich Lancaster, Fluka, or TCI-America, and were used without purification. All reaction solvents were purchased from Fisher and used as received. Reactions were monitored by TLC using pre-coated silica gel 60 F254 plates or analytical reverse phase HPLC as described in the following paragraph. Silica gel chromatography was performed with silica gel (220-240 mesh) obtained from Silicycle.

NMR spectra were recorded on a Bruker 500 MHz spectrometer. Chemical shifts are reported in δ (parts per million) by reference to the hydrogenated residues of deuterated solvent as internal standard CDCl₃ : δ = 7.28 (¹H NMR). Mass spectra were recorded on a Micromass LCT time-of-flight instrument utilizing the positive electrospray ionization mode. The purity of the compounds was assessed via analytical reverse phase HPLC with a gradient of 10 - 90% CH₃CN/water over 6 minutes (Agilent Eclipse Plus C18 4.6 × 75 mm column (3.5 µm silica), 254 nm detection).

Unless otherwise stated all temperatures are in degrees Celsius.

In these examples and elsewhere, abbreviations have the following meanings:
- NMR =: proton nuclear magnetic resonance
- aq. =: aqueous
- d =: doublet
- CH₂Cl₂ =: dichloromethane
- CH₃CN =: acetonitrile
- CH₃OH =: methanol
- CDCl₃ =: deuterited chloroform
- DIPEA =: N,N-diisopropylethylamine (iPr₂NEt)
- DMSO =: dimethyl sulfoxide
- ESI =: electrospray ionization
- EtOAc =: ethyl acetate
- Et₃N =: trimethylamine
- g =: gram
- GlcCer =: glucosyl ceramide
- h =: hours
- H₂ gas =: hydrogen gas
- HCl =: hydrochloric acid
- HBr =: hydrobromic acid
- HOAc =: acetic acid
- HOBt =: hydroxybenzotriazole
- H₂O =: water
- HATU =: 1-[bis(dimethylamino)methylene]-1H-1,2,3-triazolo[4,5-b]pyridinium 3-oxid hexafluorophosphate
- HPLC =: high performance liquid chromatography
- iPrMgCl =: isopropyl magnesium chloride
- kg =: kilogram
- K₂CO₃ =: potassium carbonate
- LiAlH₄ =: lithium aluminum hydride
- LiCl =: lithium chloride
- m =: multiplet
- mg =: milligrams
- M =: molar
- Me =: methyl
- MeOH =: methanol
- MgSO₄ =: magnesium sulfate
- MHz =: megahertz
- MsCl =: mesyl chloride
- min =: minutes
- mL =: milliliters
- mM =: millimolar
- mmol =: millimole
- ESI-MS =: mass spectrometry (electrospray ionization)
- N =: normal
- nm =: nanomolar
- N₂ =: nitrogen gas
- NaH₄Cl =: ammonium chloride
- NaI =: sodium iodide
- NaHCO₃ =: sodium bicarbonate
- NaCl =: sodium chloride
- NaOH =: sodium hydroxide
- Na₂SO₄ =: sodium sulfate
- Pd/C =: palladium on carbon
- psi =: pounds per square inch
- t_{R} =: retention time
- rt or RT =: room temperature
- s =: singlet
- satd. =: saturated
- t =: triplet
- THF =: tetrahydrofuran
- µg =: microgram
- µL =: microliter
- µmol =: micromolar
- U/mL =: units per milliliter
- UV =: ultraviolet
- v =: volume
- δ =: chemical shift

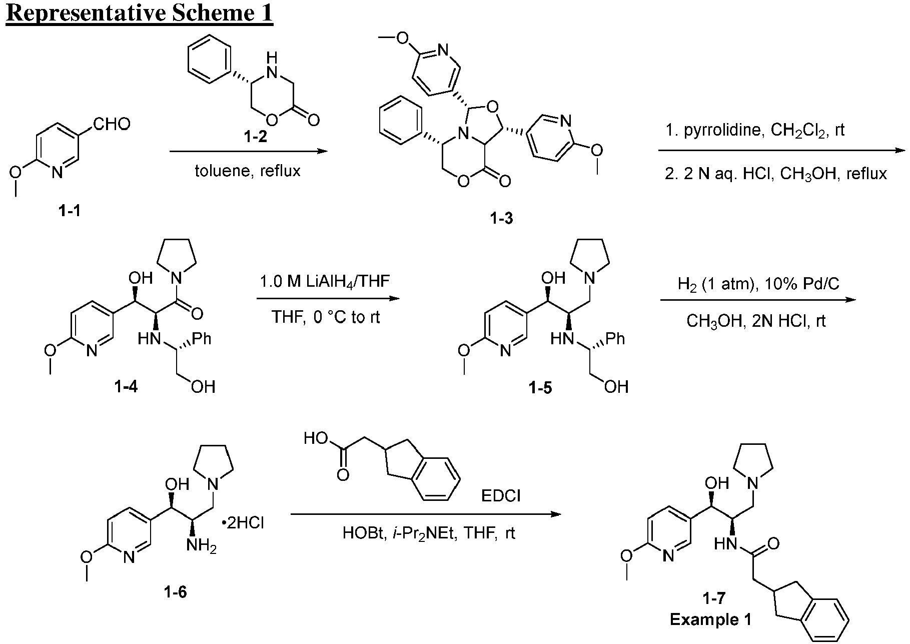

### (1R,3S,SS)-1,3-bis(6-methoxypyridin-3-yl)-5-phenyltetrahydro-3H,8H-oxazolo[4,3-c][1,4]oxazin-8-one (1-3)

(S)-5-phenylmorpholin-2-one (**1-2**, 2.2 g, 12.42 mmol) and 6-methoxy-3-nicotinaldehyde (**1-1**, 5.11 g, 37.2 mmol) were combined in toluene (60 mL). The flask was fitted with a magnetic stirrer bar, and a Dean-Stark trap. The reaction mixture was heated to reflux for 19 hours under nitrogen, (Pot temp 152 °C). Concentrated in vacuo. Purified by flash chromatography (EtOAc/ hexane). Hexane to 10% EtOAc/ hexane to 20% to 30%. (1R,3S,5S)-1,3-bis(6-methoxypyridin-3-yl)-5-phenyltetrahydro-3H,8H-oxazolo[4,3-c][1,4]oxazin-8-one (2.7 g, 6.2 mmol, 50.2 % yield). Product solidified on concentration. white solid. ¹H NMR (400 MHz, DMSO-d₆) δ 8.23 (d, J = 2.4 Hz, 1H), 8.00 (d, J = 2.3 Hz, 1H), 7.81 (dd, J = 8.6, 2.5 Hz, 1H), 7.60 (m, 1H), 7.34 - 7.07 (m, 5H), 6.89 (d, J = 8.6 Hz, 1H), 6.56 (d, J = 8.5 Hz, 1H), 5.42 (d, J = 6.6 Hz, 1H), 5.30 (s, 1H), 4.62 - 4.42 (m, 2H), 4.35 (dd, J = 11.4, 3.7 Hz, 1H), 4.19 (dd, J = 10.4, 3.7 Hz, 1H), 3.85 (s, 3H), 3.72 (s, 3H). HPLC: tᵣ 7.15min.

### (2S,3R)-3-hydroxy-2-(((R)-2-hydroxy-1-phenylethyl)amino)-3-(6-methoxypyridin-3-yl)-1-(pyrrolidin-1-yl)propan-1-one (1-4)

A solution of (1R,3S,5S)-1,3-bis(6-methoxypyridin-3-yl)-5-phenyltetrahydro-3H,8H-oxazolo[4,3-c][1,4]oxazin-8-one (**1-3**, 1.4 g, 3.23 mmol) and pyrrolidine (1.6 g, 22.6 mmol) in CH₂Cl₂ (40 mL) was stirred overnight at room temperature. Concentrated. Treated with 2 M HCl until acidic. Added MeOH and stirred at 60 °C for 2 hours. Concentrated. Basified aqueus layer with satd. aq. NaHCO₃ and partitioned between ether and water. Extracted aqueous layer (3 × EtOAc). Combined extracts, washed with satd. NaCl solution and dried, MgSO₄. Purified by flash chromatography (MeOH/ CH₂Cl₂). Obtained (3R)-3-hydroxy-2-(((S)-2-hydroxy-1-phenylethyl)amino)-3-(6-methoxypyridin-3-yl)-1-(pyrrolidin-1-yl)propan-1-one (0.9 g, 2.3 mmol, 72.3 % yield). ¹H NMR (400 MHz, Chloroform-d) δ 7.43 - 7.16 (m, 4H), 7.13 (m, 1H), 7.00 (m, 1H), 6.83 (t, J = 8.5 Hz, 1H), 4.50 (d, J = 8.6 Hz, 1H), 3.83 (s, 3H), 3.79 - 3.59 (m, 2H), 3.14 - 2.95 (m, 2H), 2.95 - 2.76 (m, 2H), 2.35 - 2.12 (m, 1H), 2.04 - 1.68 (m, 2H), 1.51 - 0.99 (m, 4H). HR-MS(ESI) m/z: Calcd for [M+H+Na]⁺ 408.190 ; Found: 408.189. HPLC: tᵣ 4.33 min.

### (1R,2R)-2-(((R)-2-hydroxy-1-phenylethyl)amino)-1-(6-methoxypyridin-3-yl)-3-(pyrrolidin-1-yl)propan-1-ol (1-5)

To a solution of (2S,3R)-3-hydroxy-2-(((R)-2-hydroxy-1-phenylethyl)amino)-3-(6-methoxypyridin-3-yl)-1-(pyrrolidin-1-yl)propan-1-one (**1-4**, 1 g, 2.3 mmol) in dry THF (20 mL) was added aluminum(III) lithium hydride (2.94 ml, 7.07 mmol). The resulting mixture was stirred overnight at room temperature. Heated to 50 °C for 1 hour. Cooled to 0 °C and treated with 0.15 ml water followed by 0.15 mL of 15% NaOH, then 0.45 ml of water. Stirred 60 minutes and filtered off solid material through celite. Concentrated. Treated with 0.2 ml 10% Citric acid. Placed under high vacuum overnight. Purified by flash chromatography (2% MeOH/ CH₂Cl₂) to 5% then 2% of 7% ammonia! methanol/ CH₂Cl₂ to 5% then 10%. Concentrated appropriate fractions to obtain 2-(((S)-2-hydroxy-1-phenylethyl)amino)-1-(6-methoxypyridin-3-yl)-3-(pyrrolidin-1-yl)propan-1-ol (0.45 g, 1.211 mmol, 46.7 % yield). ¹H NMR (400 MHz, Chloroform-d) δ 8.10 (dt, *J* = 2.5, 0.7 Hz, 1H), 7.56 (m, 1H), 7.40 - 7.14 (m, 4H), 6.73 (dd, *J* = 8.5, 0.7 Hz, 1H), 4.52 (d, *J* = 5.2 Hz, 1H), 3.93 (s, 3H), 3.79 (m, 1H), 3.62 (dd, *J* = 11.0, 4.4 Hz, 1H), 3.57 - 3.40 (m, 1H), 2.97 (m, 1H), 2.55 (dd, *J* = 12.5, 8.2 Hz, 2H), 2.37 (m, 3H), 2.30 - 2.04 (m, 1H), 1.89 - 1.31 (m, 4H). HR-MS(ESI) *m*/*z*: Calcd for [M+H]⁺ 372.221 ; Found: 372.225. *HPLC: t, 4.19 min*

### (1R,2R)-2-amino-1-(6-methoxypyridin-3-yl)-3-(pyrrolidin-1-yl)propan-1-ol (1-6)

(1R,2R)-2-(((R)-2-hydroxy-1-phenylethyl)amino)-1-(6-methoxypyridin-3-yl)-3-(pyrrolidin-1-yl)propan-1-ol (**1-5**, 0.45 g, 1.2 mmol) in MeOH (15mL) and 2M HCl (5 mL) was added 10% Pd/ C Degussa, (25mg). Nitrogen was bubbled through the solution for 5 minutes and the vessel was placed on a Parr Hydrogenator. The solution was placed under a vacuum before filling with H₂ gas. The mixture was agitated overnight under hydrogen. Placed vessel under vacuum to remove H₂ gas. Filtered mixture over celite with MeOH eluent. Concentrated and used without purification. (0.23 g, 0.915 mmol, 76 % yield).

¹H NMR (500 MHz, Chloroform-d) δ 7.62 - 7.61 (m, 1H), 7.03 - 6.71 (m, 2H), 4.59 (m, 1H), 3.79 (m, 2H), 3.14 (m, 1H), 2.75 - 2.26 (m, 5H), 1.78 (br s, 4H). HPLC: tᵣ 0.95 min.

### 2-(2,3-dihydro-1H-inden-2-yl)-N-((1R,2R)-1-hydroxy-1-(6-methoxypyridin-3-yl)-3-(pyrrolidin-1-yl)propan-2-yl)acetamide (1-7, Example 1)

To a solution of (1R,2R)-2-amino-1-(6-methoxypyridin-3-yl)-3-(pyrrolidin-1-yl)propan-1-ol (**1-6**, 0.1 g, 0.39 mmol) in dry DMF was added 1H-benzo[d][1,2,3]triazol-1-ol (0.07 g, 0.52 mmol) 3-(((ethylimino)methylene)amino)-N,N-dimethylpropan-1-amine hydrochloride (0.10 g, 0.52 mmol) and Hunigs Base. 2-(2,3-dihydro-1H-inden-2-yl)acetic acid (0.08g, 0.44 mmol) was added, and the resulting mixture was stirred overnight at room temperature. Diluted with EtOAc/ ether and washed with satd. NaCl. Purified by flash chromatography (MeOH/ CH₂Cl₂. Obtained 2-(2,3-dihydro-1H-inden-2-yl)-N-((1R,2R)-1-hydroxy-1-(6-methoxypyridin-3-yl)-3-(pyrrolidin-1-yl)propan-2-yl)acetamide (0.07 g, 0.171 mmol, 43.0 % yield. ¹H NMR (400 MHz, Chloroform-d) δ 8.19 (d, J = 2.8 Hz, 1H), 7.50 (d, J = 8.7 Hz, 1H), 7.36 - 7.21 (m, 1H), 7.21 - 6.99 (m, 3H), 6.91 (d, J = 8.1 Hz, 1H), 4.99 (d, J = 2.9 Hz, 1H), 4.55 (m, 1H), 3.84 (s, 3H), 2.95 (m, 2H), 2.85 - 2.34 (m, 8H), 2.37 - 2.07 (m, 3H), 1.78 (d, J = 3.3 Hz, 4H). HR-MS(ESI) m/z: Calcd for [M+H]⁺ 409.237 ; Found: 409.243. HPLC: tᵣ 4.5 min.

### Example 2 was prepared as described in Representative Scheme 1 for Example 1

### (1S,3S,5S)-1,3-bis(5-methoxypyridin-2-yl)-5-phenyltetrahydro-3H,8H-oxazolo[4,3-c][1,4]oxazin-8-one

(S)-5-phenylmorpholin-2-one (0.52 g, 2.92 mmol) and 5-methoxypicolinaldehyde (1, 7.29 mmol) toluene (200 mL). The flask was fitted with a magnetic stirrer bar, a Dean-Stark trap. The reaction mixture was heated to reflux for 19 hours under nitrogen, (Pot temp 152 °C). Concentrated in vacuo. Purified by flash chromatography (EtOAc/ hexane). Hexane to 10% EtOAc/ hexane to 20% to 30%. Obtained (1S,3S,5S)-1,3-bis(5-methoxypyridin-2-yl)-5-phenyltetrahydro-3H,8H-oxazolo[4,3-c][1,4]oxazin-8-one (0.65 g, 1.50 mmol, 51.4 % yield). Product solidified on concentration, white solid. Triturated in ether/ hexane. NMR, HPLC ¹H NMR (400 MHz, Chloroform-d) δ 8.32 (d, J = 2.9 Hz, 1H), 8.01 (d, J = 2.9 Hz, 1H), 7.47 (d, J = 8.6 Hz, 1H), 7.43 - 7.07 (m, 6H), 6.98 (dd, J = 8.6, 2.9 Hz, 1H), 5.70 - 5.37 (m, 2H), 4.95 (d, J = 7.2 Hz, 1H), 4.63 - 4.06 (m, 4H), 4.06 - 3.52 (m, 6H). HPLC: tᵣ 6.9 min.

### (2S,3R)-3-hydroxy-2-(((R)-2-hydroxy-1-phenylethyl)amino)-3-(5-methoxypyridin-2-yl)-1-(pyrrolidin-1-yl)propan-1-one

(1S,3S,5S)-1,3-bis(5-methoxypyridin-2-yl)-5-phenyltetrahydro-3H,8H-oxazolo[4,3-c][1,4]oxazin-8-one (0.65 g, 1.5 mmol) and pyrrolidine (0.64 g, 9.0 mmol) in CH₂Cl₂ (40 mL) was stirred overnight at room temperature. Concentrated. Treated with 2 M HCl until acidic. Added MeOH and stirred at 60 °C for 2 hours. Concentrated. Basified aqueus layer.Partitioned between ether and water. Extracted aqueous layer (3 × EtOAc). Combined extracts, washed with satd. NaCl solution and dried, MgSO₄. Purified by flash chromatography (MeOH/ CH₂Cl₂). Obtained (3S)-3-hydroxy-2-(((S)-2-hydroxy-1-phenylethyl)amino)-3-(5-methoxypyridin-2-yl)-1-(pyrrolidin-1-yl)propan-1-one (0.32 g, 0.83 mmol, 55.4 % yield). ¹H NMR (400 MHz, Chloroform-d) δ 8.32 - 8.18 (m, 1H), 7.42 (d, J = 8.6 Hz, 1H), 7.39 - 7.03 (m, 4H), 4.74 (d, J = 5.2 Hz, 2H), 4.23 - 3.95 (m, 2H), 3.85 (d, J = 2.2 Hz, 3H), 3.78 - 3.40 (m, 4H), 3.14 (m, 2H), 3.01 (m, 1H), 2.83 (m, 1H), 2.71 (m, 2H), 1.81 - 1.34 (m, 4H). HR-MS(ESI) m/z: Calcd for [M+H+Na]⁺ 409.237 ; Found: 409.243. HPLC: tᵣ 4.3 min.

### (1R,2R)-2-(((R)-2-hydroxy-1-phenylethyl)amino)-1-(5-methoxypyridin-2-yl)-3-(pyrrolidin-1 -yl)propan-1 -ol

To a 0 °C solution of (2S,3R)-3-hydroxy-2-(((R)-2-hydroxy-1-phenylethyl)amino)-3-(5-methoxypyridin-2-yl)-1-(pyrrolidin-1-yl)propan-1-one (0.34 g, 0.88 mmol) in dry THF (15 mL) was added aluminum(III) lithium hydride (0.067 g, 1.76 mmol). The resulting mixture was stirred overnight at room temperature. Heated to 50 °C for 1 hour. Cooled to 0 °C and treated with 0.15 ml water followed by 0.15 mL of 15% NaOH, then 0.45 ml of water. Stirred 60 minutes and filtered off solid material through celite. Concentrated. Treated with 0.2 ml 10% Citric acid. Placed under high vacuum overnight. Purified by flash chromatography (2% MeOH/ CH₂Cl₂) to 5% then 2% of 7% ammonia! methanol/ CH₂Cl₂ to 5% then 10%. Obtained (1S)-2-(((S)-2-hydroxy-1-phenylethyl)amino)-1-(5-methoxypyridin-2-yl)-3-(pyrrolidin-1-yl)propan-1-ol (0.14 g, 0.38 mmol, 42.7 % yield). ¹H NMR (400 MHz, Chloroform-d) δ 8.10 (m, 1H), 7.56 m, 1H), 7.39 - 7.21 (m, 8H), 6.73 (d, J = 8.6Hz, 1H), 4.52 (d, J = 5.4 Hz, 1H), 3.94 (s, 3H), 3.79 (m, 1H), 3.62 (d, J = 11.0Hz, 1H), 3.56 - 3.42 (m, 2H), 2.97 (m, 1H), 2.55 (m, 1H), 2.37 (m, 3H), 2.25 - 2.13 (m, 1H), 1.76 - 1.62 (m, 3H). HR-MS(ESI) m/z: Calcd for [M+H]⁺ 372.221 ; Found: 372.225. HPLC: tᵣ 4.5 min.

### (1R,2R)-2-amino-1-(5-methoxypyridin-2-yl)-3-(pyrrolidin-1-yl)propan-1-ol

To a solution of (1R,2R)-2-(((R)-2-hydroxy-1-phenylethyl)amino)-1-(5-methoxypyridin-2-yl)-3-(pyrrolidin-1-yl)propan-1-ol (0.45 g, 1.21 mmol) in MeOH (15mL) and 2M HCl (5 mL) was added 10% Pd/ C Degussa, (25mg). Nitrogen was bubbled through the solution for 5 minutes and the vessel was placed on a Parr Hydrogenator. The solution was placed under a vacuum before filling with H₂ gas. The mixture was agitated overnight under hydrogen. Placed vessel under vacuum to remove H₂ gas. Filtered mixture over celite with MeOH eluent. Concentrated and used without purification. (1S)-2-amino-1-(5-methoxypyridin-2-yl)-3-(pyrrolidin-1-yl)propan-1-ol (0.08g, 0.32 mmol, 84%) oil.

¹H NMR (400 MHz, Chloroform-d) δ 8.36 - 7.99 (m, 1H), 7.58 (dd, J = 8.5, 2.4 Hz, 1H), 6.74 (d, J = 8.5 Hz, 1H), 4.62 (d, J = 3.9 Hz, 1H), 3.93 (d, J = 0.6 Hz, 3H), 3.13 (m, 1H), 2.81 - 2.33 (m, 7H), 1.77 (m, 4H). HPLC: tᵣ 1.0 min.

### 2-(2,3-dihydro-1H-inden-2-yl)-N-((1R,2R)-1-hydroxy-1-(5-methoxypyridin-2-yl)-3-(pyrrolidin-1-yl)propan-2-yl)acetamide (Example 2)

To a solution of (1R,2R)-2-amino-1-(5-methoxypyridin-2-yl)-3-(pyrrolidin-1-yl)propan-1-ol (0.1 g, 0.39 mmol) in dry DMF was added 1H-benzo[d][1,2,3]triazol-1-ol (0.070 g, 0.52 mmol) 3-(((ethylimino)methylene)amino)-N,N-dimethylpropan-1-amine hydrochloride (0.10 g, 0.52 mmol) and Hunigs Base. 2-(2,3-dihydro-1H-inden-2-yl)acetic acid (0.08g, 0.44 mmol) was added, and the resulting mixture was stirred overnight at room temperature. Diluted with EtOAc/ ether and washed with satd. NaCl. Purified by flash chromatography (MeOH/ CH₂Cl₂. Obtained 2-(2,3-dihydro-1H-inden-2-yl)-N-((1R,2R)-1-hydroxy-1-(6-methoxypyridin-3-yl)-3-(pyrrolidin-1-yl)propan-2-yl)acetamide (0.05 g, 0.122 mmol, 30.7 % yield). ¹H NMR (400 MHz, Chloroform-d) δ 7.89 (dd, J = 6.3, 3.1 Hz, 1H), 7.44 (dd, J = 6.3, 3.0 Hz, 1H), 7.38 - 6.93 (m, 7H), 3.34 - 3.09 (m, 5H), 3.08 - 2.77 (m, 5H), 2.77 - 2.26 (m, 7H), 1.42 (d, J = 6.7 Hz, 1H), 1.29 - 1.01 (m, 5H). HPLC: tᵣ 4.5 min.

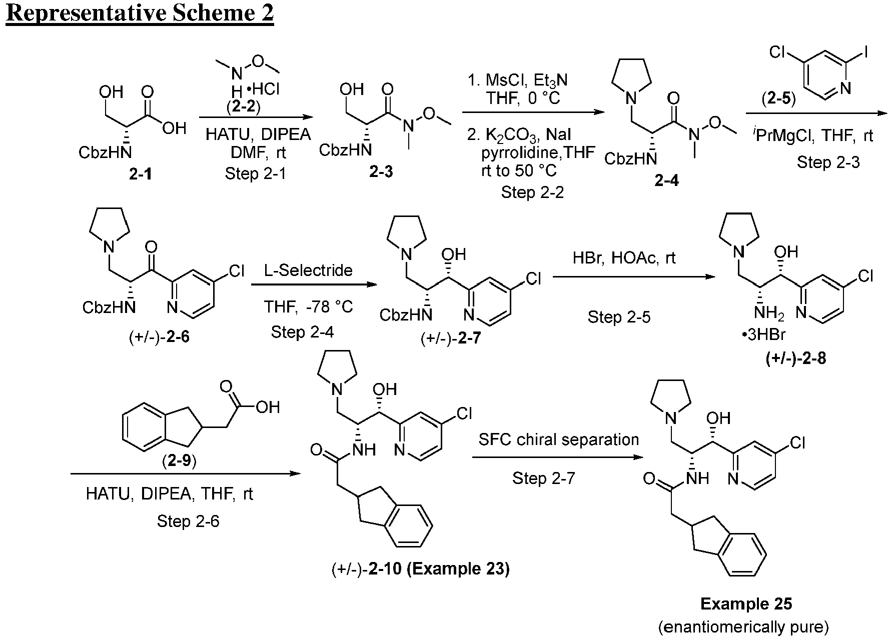

### Preparation of (R)-benzyl (3-hydroxy-1-(methoxy(methyl)amino)-1-oxopropan-2-yl)carbamate (2-3, Step 2-1).

To a stirred solution of compound **2-1** (5.00 g, 20.9 mmol), compound **2-2** (6.11 g, 62.6 mmol), and DIPEA (18.2 mL, 104 mmol) in anhydrous DMF (50 mL) was added HATU (8.74 g, 23.0 mmol) at rt under nitrogen. The resulting mixture was stirred for overnight. The reaction mixture was then quenched with water (100 mL) and extracted with EtOAc (3 ×). The combined extracts were washed with 10% LiCl aqueous solution (3 ×), dried over anhydrous Na₂SO₄, filtered, and concentrated under reduced pressure. The resulting residue was further dried under high vacuum to afford compound **2-3** as a colorless syrup (5.24 g, 88%): ESI MS, *m*/*z* = 283 [M+H]⁺.

### Preparation of (R)-benzyl (1-(methoxy(methyl)amino)-1-oxo-3-(pyrrolidin-1-yl)propan-2-yl)carbamate (2-4, Step 2-2).

To a stirred solution of compound **2-3** (3.50 g, 12.4 mmol) and Et₃N (5.2 mL, 37.3 mmol) in anhydrous THF (50 mL) was added MsCl (1.4 mL, 18.1 mmol) dropwise at 0 °C under nitrogen. The resulting mixture was stirred for 30 min at 0 °C. LC/MS indicated the reaction finished. The reaction mixture was then diluted with EtOAc and quenched with water. The layers were separated and the aqueous layer was back extracted with EtOAc (3 ×). The combined organic layers were washed with brine, dried over anhydrous Na₂SO₄, filtered, and concentrated under reduced pressure. The resulting residue was further dried under high vacuum and dissolved in anhydrous THF (50 mL). To the resulting solution was added K₂CO₃ (3.43 g, 24.8 mmol), NaI (1.86 g, 12.4 mmol), and pyrrolidine (2.1 mL, 25.2 mmol) at rt under nitrogen. The reaction mixture was then heated at 50 °C for 1.5 h. After this time, the reaction mixture was cooled to rt and filtered. The filter cake was washed with EtOAc. The filtrate was concentrated under reduced pressure. The resulting residue was purified by flash column chromatography on silica gel eluting with 2% to 5% MeOH/CH₂Cl₂ to afford compound **2-4** as a dark red syrup (1.57 g, 38%.): ESI MS, *m*/*z* = 336 [M+H]⁺.

### Preparation of (+/-)-benzyl (1-(4-chloropyridin-2-yl)-1-oxo-3-(pyrrolidin-1-yl)propan-2-yl)carbamate (2-6, Step 2-3).

To a stirred mixture of compound **2-4** (2.73 g, 8.14 mmol) and compound **2-5** (2.14 g, 8.94 mmol) in anhydrous THF (60 mL) was added 2M *ⁱ*PrMgCl solution in THF (16.3 mL, 32.6 mmol) dropwise over 60 min at rt under N₂. The resulting mixture was stirred for 60 min. After this time, the reaction mixture was quenched with brine and extracted with EtOAc (3 ×). The combined extracts were dried over anhydrous Na₂SO₄, filtered, and concentrated under reduced pressure. The resulting residue was further dried under high vacuum to afford crude compound **2-6** as an orange oil (3.10 g) which was carried over to the next step without further purification: ESI MS, *m*/*z* = 388 [M+H]⁺.

### Preparation of (+/-)-benzyl (1-(4-chloropyridin-2-yl)-1-hydroxy-3-(pyrrolidin-1-yl)propan-2-yl)carbamate (2-7, Step 2-4).

To a stirred solution of crude compound **2-6** (3.10 g) in anhydrous THF (120 mL) was added 1M L-Selectride solution in THF (16.0 mL, 16.0 mmol) dropwise at -78 °C under N₂. The resulting mixture was stirred at -78 °C for 2 h and then quenched with saturated NH₄Cl aqueous solution, and extracted with EtOAc (3 ×). The combined extracts were dried over anhydrous Na₂SO₄, filtered, and concentrated under reduced pressure. The resulting residue was purified by flash column chromatography on silica gel eluting with 5% to 50% (80:18:2 = CH₂Cl₂/MeOH/NH₄OH)/CH₂Cl₂ to afford compound **2-7** as a brown foam (1.50 g, 47% over two steps.): ESI MS, *m*/*z* = 390 [M+H]⁺.

### Preparation of (+/-)-2-amino-1-(4-chloropyridin-2-yl)-3-(pyrrolidin-1-yl)propan-1-ol hydrobromide (2-8, Step 2-5).

To a stirred solution of compound **2-7** (1.37 g, 3.52 mmol) in HOAc (50 mL) was added a 33% HBr solution in HOAc (8 mL) dropwise over 20 min at rt. The reaction mixture was stirred at rt for 6 h, then concentrated under reduced pressure. The resulting residue was further dried under high vacuum to afford crude compound **2-8** as a brown foam (1.75 g) which was carried to the next step without further purification: ESI MS, *m*/*z* = 256 [M+H]⁺.

### Preparation of (+/-)-N-(1-(4-chloropyridin-2-yl)-1-hydroxy-3-(pyrrolidin-1-yl)propan-2-yl)-2-(2,3-dihydro-1H-inden-2-yl)acetamide (2-10, Step 2-6) (Example 23).

To a stirred solution of crude compound **2-8** (1.75 g), compound **2-9** (618 mg, 3.51 mmol), and DIPEA (6.11 mL, 35.1 mmol) in anhydrous THF (80 mL) was added HATU (1.33 g, 3.50 mmol). The reaction mixture was stirred for 1 h. After this time, the reaction was quenched with brine and extracted with EtOAc (3 ×). The combined extracts were dried over anhydrous Na₂SO₄, filtered, and concentrated under reduced pressure. The resulting residue was purified by flash column chromatography on silica gel eluting with 5% to 40% (80:18:2 = CH₂Cl₂/MeOH/NH₄OH)/CH₂Cl₂ to afford compound **2-10** as a white solid (720 mg, 50% over two steps): ESI MS, *m*/*z* = 414 [M+H]⁺. ¹H NMR (500 MHz, DMSO-*d*₆) δ 8.47 (d, *J* = 5.0 Hz, 1H), 7.53 (d, *J* = 2.0 Hz, 1H), 7.48 (bs, 1H), 7.40 (d, *J* = 3.5 Hz, 1H), 7.15-7.05 (m, 4H), 5.89 (bs, 1H), 4.91 (s, 1H), 4.34 (bs, 1H), 2.83 (bs, 1H), 2.76-2.42 (m, 7H), 2.37-2.08 (m, 5H), 1.71 (bs, 4H); ESI MS, *m*/*z* = 414 [M+H]⁺.

Preparation of *N*-((1*S*,2*R*)-1-(4-chloropyridin-2-yl)-1-hydroxy-3-(pyrrolidin-1-yl)propan-2-yl)-2-(2,3-dihydro-1*H*-inden-2-yl)acetamide (Example 25, EEF-AN-21, Step 2-7).

SFC chiral separation of compound (+/-)-**2-10**:
Analytical SFC method for compound (+/-)**-2-10**: Chiralpak @ OD-H (1.6 × 100 mm, 5 micron); mobile phase: 20% MeOH in CO₂; 1.5 ml/min; 8 min at 40 °C; 1750 psi; @ 220 nm; injection: 4 uL of a 1 mg/ml solution in MeOH; retention time (tᵣ): 2.94 min (**Example 25**) and 3.57 min (**Example 26**).

Preparative SFC chiral separation of compound (+/-)-**2-10** (145 mg): ChiralCel^{®} OD-H (30 × 250 mm, 5 micron); mobile phase: 20% MeOH in CO₂; 100 g/min at 40 °C; 120 bar; @ 220 nm; injection: 0.7 mL of a 0.1 M solution in MeOH. After separation from SFC, the resulting residue (after concentration under reduced pressure) containing desired enantiomer was further purified by silica gel column chromatography eluting with 0% to 80% (80:18:2 = CH₂Cl₂/MeOH/NH₄OH)/CH₂Cl₂ to afford **Example 25** (48 mg, 33%) as a white solid: ESI MS, *m*/*z* = 414 [M+H]⁺.

**Example 25:** ¹H NMR (300 MHz, CDCl₃) δ 8.40 (d, *J* = 5.4 Hz, 1H),7.66 (d, *J* = 2.0 Hz, 1H), 7.25-7.23 (m, 1H), 7.18-7.11 (m, 4H), 7.00-6.96 (m, 1H), 5.02-5.00 (m, 1H), 4.61-4.58 (m, 1H), 3.08-2.91 (m, 2H), 2.88-2.40 (m, 9H), 2.36-2.19 (m, 2H), 1.86-1.73 (m, 4H); ESI MS *m*/*z* 414 [M+H]⁺.

**Example 26:** ¹H NMR (300 MHz, CDCl₃) δ 8.40 (d, *J* = 6.0 Hz, 1H),7.67 (d, *J* = 2.1 Hz, 1H), 7.25-7.22 (m, 1H), 7.18-7.10 (m, 4H), 7.00-6.97 (m, 1H), 5.02-5.00 (m, 1H), 4.64-4.56 (m, 1H), 3.08-2.91 (m, 2H), 2.88-2.40 (m, 9H), 2.36-2.19 (m, 2H), 1.86-1.73 (m, 4H); ESI MS *m*/*z* 414 [M+H]⁺.

The following Examples were prepared by the synthetic route depicted in Representative Scheme 2 using the same procedures as described for Examples 23, 25 and 26. Typically, the final product was isolated as a racemate, but in some cases the racemate was separated by chiral chromatography (as described for Examples 25 and 26) into the individual enantiomers.

### 2-(2,3-dihydro-1H-inden-2-yl)-N-((1S,2R)-1-hydroxy-1-(6-methoxypyridin-2-yl)-3-(pyrrolidin-1-yl)propan-2-yl)acetamide (Example 3)

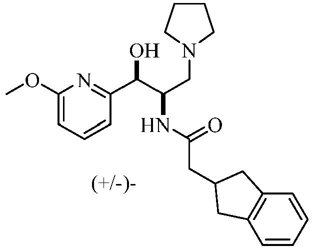

¹H NMR (500 MHz, DMSO-*d*₆) δ 9.1 (s, 1H), 7.78 (d, *J* = 9.5 Hz, 1H), 7.71 (t, *J* = 7.5 Hz, 1H), 7.11-7.05 (m, 5H), 6.71 (d, *J* = 8.5 Hz, 1H), 4.79-4.77 (m, 2H), 3.87 (s, 3H), 3.65-3.50 (m, 3H), 3.36-3.25 (m, 1H), 3.16-3.07 (m, 2H), 2.75-2.70 (m, 1H), 2.55-2.45 (m, 2H), 2.39-2.36 (m, 1H), 2.18-2.09 (m, 3H), 2.01-1.80 (m, 4H); ESI MS, *m*/*z* = 410 [M+H]⁺.

### 2-(2,3-dihydro-1H-inden-2-yl)-N-((1S,2R)-1-hydroxy-1-(pyridin-2-yl)-3-(pyrrolidin-1-yl)propan-2-yl)acetamide (Example 4)

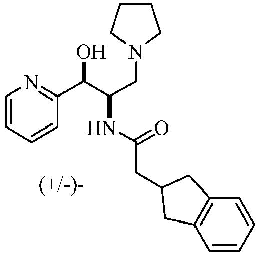

¹H NMR (500 MHz, DMSO-d₆) δ 8.47 (bs, 1H), 7.73 (dd, *J* = 1.5, 7.5 Hz, 1H), 7.47 (d, *J* = 8.0 Hz, 1H), 7.38 (d, *J* = 9.0 Hz, 1H), 7.23 (m, 1H), 7.12-7.06 (m, 4H), 5.68 (d, *J* = 4.5 Hz, 1H), 4.87 (s, 1H), 4.31 (m, 1H), 2.80-2.72 (m, 2H), 2.63-2.45 (m, 6H), 2.38-2.32 (m, 2H), 2.24-2.09 (m, 3H), 1.69 (bs, 4H); ESI MS, *m*/*z* = 380 [M+H]⁺.

### 2-(2,3-dihydro-1H-inden-2-yl)-N-((1S,2R)-1-hydroxy-3-(pyrrolidin-1-yl)-1-(5-(trifluoromethoxy)pyridin-2-yl)propan-2-yl)acetamide (Example 5)

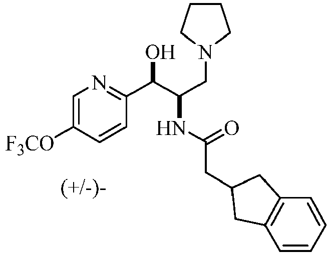

¹H NMR (500 MHz, DMSO-d₆) δ 8.57 (d, *J* = 2.8 Hz, 1H), 7.85 (dd, *J* = 1.6, 8.6 Hz, 1H), 7.60 (d, *J* = 8.6 Hz, 1H), 7.46 (d, *J* = 9.2 Hz, 1H), 7.11-7.06 (m, 4H), 5.90 (bs, 1H), 4.93 (d, *J* = 1.8 Hz, 1H), 4.35-4.28 (m, 1H), 2.85-2.45 (m, 7H), 2.40-2.05 (m, 5H), 1.69 (bs, 4H); ESI MS, *m*/*z* = 464 [M+H]⁺.

### 2-(2,3-dihydro-1H-inden-2-yl)-N-((1S,2R)-1-hydroxy-3-(pyrrolidin-1-yl)-1-(5-(2,2,2-trifluoroethoxy)pyridin-2-yl)propan-2-yl)acetamide (Example 6)

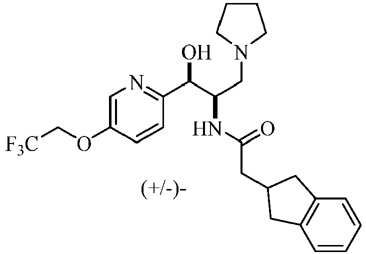

¹H NMR (500 MHz, DMSO-*d*₆) δ 8.30 (d, *J* = 2.7 Hz, 1H), 7.50-7.46 (m, 1H), 7.45-7.41 (m, 1H), 7.39-7.34 (m, 1H), 7.13-7.05 (m, 4H), 5.68-5.64 (m, 1H), 4.87-4.76 (m, 3H), 4.30-4.22 (m, 1H), 2.76-2.71 (m, 2H), 2.68-2.60 (m, 1H), 2.57-2.45 (m, 4H), 2.43-2.23 (m, 3H), 2.14-2.06 (m, 2H), 1.74-1.64 (m, 4H); ESI MS *m*/*z* 478 [M+H]⁺.

### N-((1S,2R)-1-(5-(tert-butoxy)pyridin-2-yl)-1-hydroxy-3-(pyrrolidin-1-yl)propan-2-yl)-2-(2,3-dihydro-1H-inden-2-yl)acetamide (Example 7)

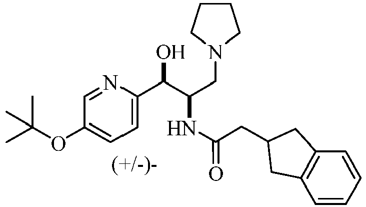

¹H NMR (500 MHz, DMSO-*d*₆) δ 8.14 (s, 1H), 7.38-7.36 (m, 3H), 7.12-7.11 (m, 4H), 5.80 (bs, 1H), 4.85 (d, *J* = 2.5 Hz, 1H), 4.28-4.22 (m, 1H), 2.78-2.74 (m, 2H), 2.68-2.62 (m, 1H), 2.54-2.43 (m, 4H), 2.31-2.29 (m, 4H), 2.11-2.08 (m, 2H), 1.69 (bs, 4H), 1.28 (s, 9H); ESI MS, *m*/*z* = 452 [M+H]⁺.

### 2-(2,3-dihydro-1H-inden-2-yl)-N-((1R,2R)-1-hydroxy-1-(2-methoxypyridin-4-yl)-3-(pyrrolidin-1-yl)propan-2-yl)acetamide (Example 8)

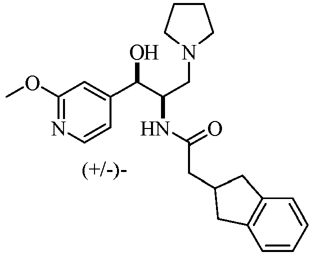

¹H NMR (500 MHz, DMSO-d₆) δ 8.04 (d, *J* = 5.5 Hz, 1H), 7.50 (d, *J* = 9.0 Hz, 1H), 7.12-7.06 (m, 4H), 6.90 (dd, *J* = 1.0, 5.5 Hz, 1H), 6.75 (s, 1H), 5.75 (bs, 1H), 4.82 (s, 1H), 4.16-4.10 (m, 1H), 3.81 (s, 3H), 2.77-2.63 (m, 3H), 2.54-2.46 (m, 3H), 2.40-2.12 (m, 6H), 1.69 (bs, 4H); ESI MS, *m*/*z* = 410 [M+H]⁺.

### 2-(2,3-dihydro-1H-inden-2-yl)-N-((1R,2R)-1-hydroxy-1-(5-methoxypyridin-3-yl)-3-(pyrrolidin-1-yl)propan-2-yl)acetamide (Example 9)

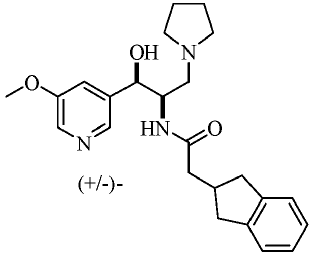

¹H NMR (500 MHz, DMSO-*d*₆) δ 8.15-8.11 (m, 2H), 7.56 (d, *J* = 9.2 Hz, 1H), 7.30-7.28 (m, 1H), 7.14-7.04 (m, 4H), 5.76-5.71 (m, 1H), 4.92-4.89 (m, 1H), 4.17-4.09 (m, 1H), 3.78 (s, 3H), 2.83-2.70 (m, 2H), 2.69-2.60 (m, 1H), 2.50-2.48 (m, 4H), 2.47-2.38 (m, 1H), 2.37-2.25 (m, 2H), 2.19-2.11 (m, 2H), 1.73-1.46 (m, 4H); ESI MS *m*/*z* 410 [M+H]⁺.

### 2-(2,3-dihydro-1H-2-(2,3-dihydro-1H-inden-2-yl)-N-((1S,2R)-1-hydroxy-1-(5-methoxypyridin-2-yl)-3-(pyrrolidin-1-yl)propan-2-yl)-N-methylacetamide (Example 10)

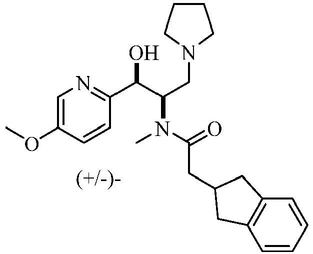

¹H NMR (500 MHz, DMSO-d₆) δ 8.15-8.05 (m, 1H), 7.36-7.33 (m, 2H), 7.15-7.06 (m, 4H), 5.61-4.11 (m, 3H), 3.78-3.75 (m, 3H), 3.00-2.53 (m, 7H), 2.49-2.10 (m, 9H), 1.66-1.55 (m, 4H); ESI MS, *m*/*z* = 424 [M+H]⁺

### 2-(2,3-dihydro-1H-inden-2-yl)-N-((1R,2S)-1-hydroxy-1-(5-methoxypyridin-2-yl)-3-(pyrrolidin-1-yl)propan-2-yl)acetamide (Example 11)

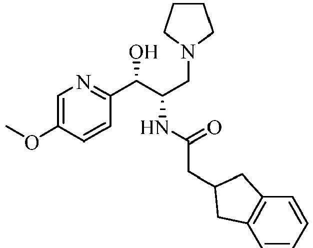

¹H NMR (500 MHz, DMSO-d₆) δ 8.19-8.18 (m, 1H), 7.40-7.31 (m, 3H), 7.12-7.06 (m, 4H), 5.61 (bs, 1H), 4.83 (s, 1H), 4.30-4.20 (m, 1H), 3.80 (s, 3H), 2.77-2.61 (m, 4H), 2.54-2.46 (m, 2H), 2.41-2.08 (m, 6H), 1.72-1.65 (m, 4H); ESI MS, *m*/*z* = 410 [M+H]⁺

### 2-(2,3-dihydro-1H-inden-2-yl)-N-((1S,2R)-1-hydroxy-1-(4-methoxypyridin-2-yl)-3-(pyrrolidin-1-yl)propan-2-yl)acetamide (Example 12)

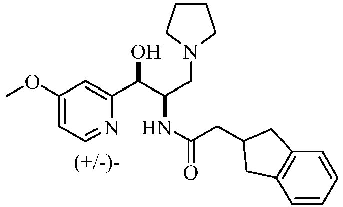

¹H NMR (500 MHz, DMSO-*d*₆) δ 8.29 (s, 1H), 7.39 (d, *J* = 9.0 Hz, 1H), 7.11-7.04 (m, 5H), 6.82 (m, 1H), 5.72 (d, *J* = 5.0 Hz, 1H), 4.84-4.83 (m, 1H), 4.33-4.29 (m, 1H), 3.77 (s, 3H), 2.80-2.70 (m, 2H), 2.57-2.54 (m, 3H), 2.50-2.47 (m, 2H), 2.46-2.25 (m, 3H), 2.14-2.07 (m, 2H), 1.69 (bs, 4H); ESI MS, *m*/*z* = 410 [M+H]⁺.

### 2-(2,3-dihydro-1H-inden-2-yl)-N-((1R,2R)-1-hydroxy-1-(pyridin-3-yl)-3-(pyrrolidin-1-yl)propan-2-yl)acetamide (Example 13)

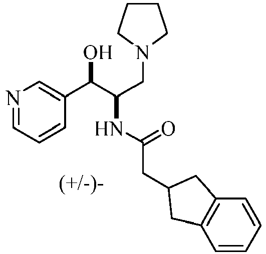

¹H NMR (500 MHz, DMSO-*d*₆) δ 8.52 (d, *J* = 2.1 Hz, 1H), 8.42 (dd, *J* = 1.6, 4.7 Hz, 1H), 7.71-7.67 (m, 1H), 7.55 (d, *J* = 9.1Hz, 1H), 7.30 (dd, *J* = 4.7, 7.7 Hz, 1H), 7.16-7.05 (m, 4H), 5.73-5.69 (m, 1H), 4.91-4.88 (m, 1H), 4.17-4.10 (m, 1H), 2.82-2.61 (m, 3H), 2.57-2.49 (m, 5H), 2.46-2.39 (m, 1H), 2.36-2.24 (m, 2H), 2.14 (d, *J* = 7.5 Hz, 2H), 1.73-1.63 (m, 4H); ESI MS *m*/*z* 380 [M+H]⁺.

### 2-(2,3-dihydro-1H-inden-2-yl)-N-((1S,2R)-1-hydroxy-1-(3-methoxypyridin-2-yl)-3-(pyrrolidin-1-yl)propan-2-yl)acetamide (Example 14)

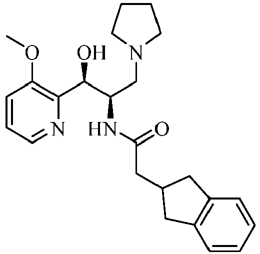

¹H NMR (500 MHz, DMSO-d₆) δ 8.07 (d, *J* = 4.0 Hz, 1H), 7.42 (d, *J* = 8.0 Hz, 1H), 7.38-7.25 (m, 2H), 7.16-7.04 (m, 4H), 5.08 (s, 1H), 4.78 (bs, 1H), 4.47 (bs, 1H), 3.85 (s, 3H), 2.83-2.72 (m, 2H), 2.62-2.33 (m, 7H), 2.24-2.15 (m, 2H), 2.01 (d, *J* = 7.0 Hz, 2H), 1.71 (bs, 4H); ESI MS, *m*/*z* = 410 [M+H]⁺.

### N-((1S,2R)-3-(3-azabicyclo[3.1.0]hexan-3-yl)-1-hydroxy-1-(5-methoxypyridin-2-yl)propan-2-yl)-2-(2,3-dihydro-1H-inden-2-yl)acetamide (Example 15)

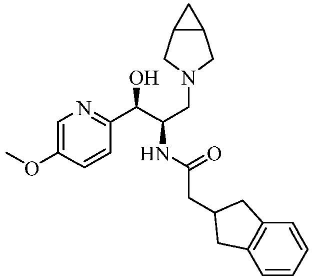

¹H NMR (500 MHz, DMSO-*d*₆) δ 8.18 (d, *J* = 2.7 Hz, 1H), 7.39-7.29 (m, 3H), 7.14-7.05 (m, 4H), 5.53 (d, *J* = 5.2 Hz, 1H), 4.77-4.73 (m, 1H), 4.22-4.14 (m, 1H), 3.79 (s, 3H), 3.05-2.97 (m, 2H), 2.79-2.71 (m, 1H), 2.70-2.60 (m, 2H), 2.43-2.22 (m, 5H), 2.14-2.05 (m, 2H), 1.39-1.28 (m, 2H), 0.59-0.54 (m, 1H), 0.32-0.26 (m, 1H); ESI MS *m*/*z* 421 [M+H]⁺.

### 2-(2,3-dihydro-1H-inden-2-yl)-N-((1S,2R)-3-(3,3-dimethylpyrrolidin-1-yl)-1-hydroxy-1-(5-methoxypyridin-2-yl)propan-2-yl)acetamide (Example 16)

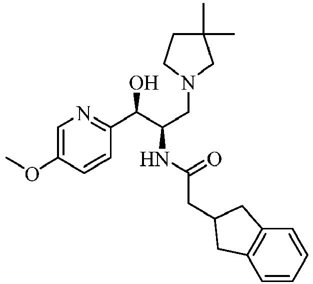

¹H NMR (500 MHz, DMSO-*d*₆) δ 8.19 (d, *J* = 3.0 Hz, 1H), 7.39 (d, *J* = 8.5 Hz, 1H), 7.33 (m, 2H), 7.13-7.06 (m, 4H), 5.60 (bs, 1H), 4.83 (s, 1H), 4.22 (bs, 1H), 3.80 (s, 3H), 3.00-2.95 (m, 2H), 2.75-2.55 (m, 4H), 2.44-2.22 (m, 5H), 2.15-2.05 (m, 2H), 1.50 (bs, 2H), 1.05 (bs, 6H); ESI MS, *m*/*z* = 438 [M+H]⁺.

### N-((1S,2R)-1-(5-(tert-butoxy)pyridin-2-yl)-1-hydroxy-3-(pyrrolidin-1-yl)propan-2-yl)-2-(2,3-dihydro-1H-inden-2-yl)acetamide (Example 17)

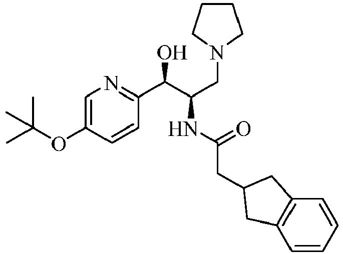

¹H NMR (500 MHz, DMSO-*d*₆) δ 8.14 (dd, *J* = 1.0, 2.4 Hz, 1H), 7.39-7.35 (m, 3H), 7.12-7.06 (m, 4H), 5.65 (bs, 1H), 4.85 (s, 1H), 4.28-4.18 (m, 1H), 2.80-2.50 (m, 5H), 2.49-2.25 (m, 6H), 2.12-2.02 (m, 2H), 1.69 (bs, 4H), 1.29 (s, 9H); ESI MS, *m*/*z* = 452 [M+H]⁺.

### N-((1R,2S)-1-(5-(tert-butoxy)pyridin-2-yl)-1-hydroxy-3-(pyrrolidin-1-yl)propan-2-yl)-2-(2,3-dihydro-1H-inden-2-yl)acetamide (Example 18)

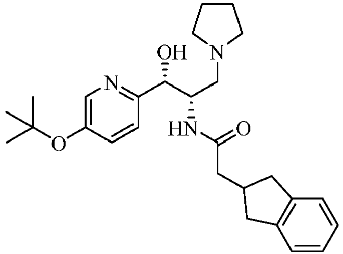

¹H NMR (500 MHz, DMSO-*d*₆) δ 8.16-8.15 (m, 1H), 7.55-7.40 (m, 3H), 7.12-7.07 (m, 4H), 5.80 (bs, 1H), 4.83 (s, 1H), 4.40 (bs, 1H), 3.10-2.50 (m, 7H), 2.49-2.55 (m, 6H), 1.75 (bs, 4H), 1.29 (s, 9H); ESI MS, *m*/*z* = 452 [M+H]⁺.

### 2-(2,3-dihydro-1H-inden-2-yl)-N-((1S,2R)-3-((R)-3-fluoropyrrolidin-1-yl)-1-hydroxy-1-(5-methoxypyridin-2-yl)propan-2-yl)acetamide (Example 19)

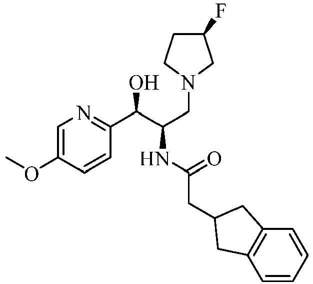

¹H NMR (500 MHz, DMSO-*d*₆) δ 8.19 (d, *J* = 2.7 Hz, 1H), 7.43-7.36 (m, 2H), 7.35-7.31 (m, 1H), 7.13-7.03 (m, 4H), 5.60 (bs, 1H), 5.27-5.10 (m, 1H), 4.84-4.81 (m, 1H), 4.29-4.18 (m, 1H), 3.80 (s, 3H), 3.30-3.23 (m, 1H), 2.92-2.70 (m, 4H), 2.69-2.51 (m, 2H), 2.44-2.31 (m, 3H), 2.30-2.22 (m, 1H), 2.18-2.03 (m, 3H), 197-1.80 (m, 1H); ESI MS *m*/*z* 428 [M+H]⁺.

### N-((1S,2R)-1-(6-chloropyridin-2-yl)-1-hydroxy-3-(pyrrolidin-1-yl)propan-2-yl)-2-(2,3-dihydro-1H-inden-2-yl)acetamide (Example 20)

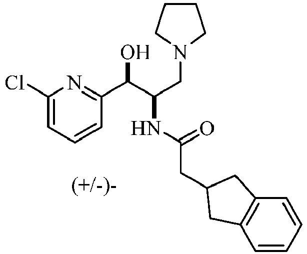

¹H NMR (500 MHz, DMSO-*d*₆) δ 7.80 (t, *J* = 7.5 Hz, 1H), 7.45 (d, *J* = 7.5 Hz, 2H), 7.35 (d, *J* = 8.0 Hz, 1H), 7.12-7.08 (m, 4H), 5.84 (bs, 1H), 4.82 (s, 1H), 4.26 (bs, 1H), 2.83-2.61 (m, 4H), 2.57-2.40 (m, 2H), 2.38-2.28 (m, 4H), 2.26-2.11 (m, 2H), 1.70 (bs, 4H); ESI MS, *m*/*z* = 414 [M+H]⁺.

### 2-(2,3-dihydro-1H-inden-2-yl)-N-((1S,2R)-1-hydroxy-3-(pyrrolidin-1-yl)-1-(5-(trifluoromethoxy)pyridin-2-yl)propan-2-yl)acetamide (Example 21)

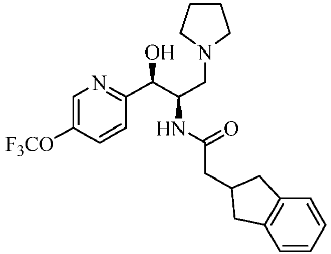

¹H NMR (500 MHz, DMSO-*d*₆) δ 8.56 (d, *J* = 2.7, 1H), 7.86-7.82 (m, 1H), 7.60 (d, *J* = 8.6 Hz, 1H), 7.45 (d, *J* = 9.2 Hz, 1H), 7.12-7.04 (m, 4H), 5.87-5.83 (m, 1H), 4.95-4.90 (m, 1H), 4.35-4.27 (m, 1H), 2.84-2.71 (m, 2H), 2.66-2.59 (m, 1H), 2.58-2.52 (m, 2H), 2.51-2.42 (m, 2H), 2.41-2.34 (m, 1H), 2.33-2.27 (m, 1H), 2.26-2.17 (m, 1H), 2.14-2.04 (m, 2H), 1.73-1.62 (m, 4H); ESI MS *m*/*z* 464 [M+H]⁺.

### 2-(2,3-dihydro-1H-inden-2-yl)-N-((1R,2S)-1-hydroxy-3-(pyrrolidin-1-yl)-1-(5-(trifluoromethoxy)pyridin-2-yl)propan-2-yl)acetamide (Example 22)

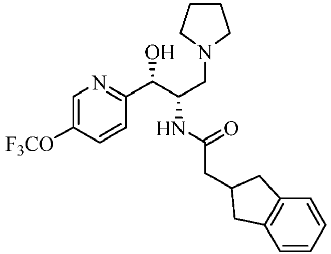

¹H NMR (500 MHz, DMSO-*d*₆) δ 8.56 (d, *J* = 2.7, 1H), 7.87-7.82 (m, 1H), 7.60 (d, *J* = 8.6 Hz, 1H), 7.46 (d, *J* = 9.3 Hz, 1H), 7.12-7.05 (m, 4H), 5.87-5.83 (m, 1H), 4.95-4.91 (m, 1H), 4.35-4.28 (m, 1H), 2.84-2.71 (m, 2H), 2.66-2.52 (m, 3H), 2.51-2.43 (m, 2H), 2.42-2.34 (m, 1H), 2.33-2.27 (m, 1H), 2.26-2.19 (m, 1H), 2.15-2.03 (m, 2H), 1.72-1.64 (m, 4H); ESI MS *m*/*z* 464 [M+H]⁺.

### N-((1S,2R)-1-(5-(tert-butoxy)pyridin-2-yl)-1-hydroxy-3-(pyrrolidin-1-yl)propan-2-yl)-2-(5-fluoro-2,3-dihydro-1H-inden-2-yl)acetamide (Example 24)

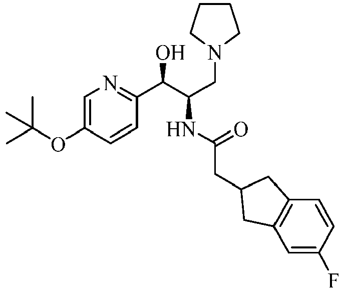

¹H NMR (500 MHz, DMSO-*d*₆) δ 8.14 (d, J= 2.0 Hz, 1H), 7.45-7.30 (m, 3H), 7.15-6.85 (m, 3H), 5.65 (bs, 1H), 4.84 (s, 1H), 4.29 (bs, 1H), 2.80-2.53 (m, 6H), 2.48-2.19 (m, 4H), 2.15-2.00 (m, 2H), 1.71 (bs, 4H), 1.28 (s, 9H); ESI MS, *m*/*z* = 470 [M+H]⁺.

### N-((1R,2R)-1-(2-chloropyridin-4-yl)-1-hydroxy-3-(pyrrolidin-1-yl)propan-2-yl)-2-(2,3-dihydro-1H-inden-2-yl)acetamide (Example 27)

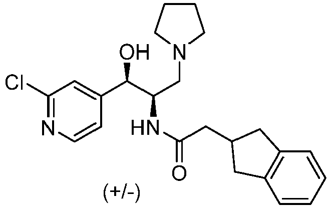

¹H NMR (400 MHz, CDCl₃) δ 8.38 (d, *J* = 5 Hz, 1H), 7.42 (s, 1H), 7.13 - 7.22 (m, 5H), 5.91 (d, *J* = 7.6 Hz, 1H), 5.12 (d, J = 2 Hz, 1H), 4.33 (m, 1H), 2.8 - 3.2 (m, 9H), 2.51 (dd, J = 6, 16 Hz, 1H), 2.31 (m, 2H), 2.18 (dd, *J* = 8, 14 Hz, 1H), 1.84 (m, 5H). ESI MS, *m*/*z* = 414.1 [M+H]⁺.

### GCS Inhibition

GCS inhibitors are known. Some GCS inhibitors, e.g., eliglustat and miglustat, possess sufficient activity to inhibit GCS activity, and therefore have been proposed as suitable for treating diseases related to glycolipid accumulation. However, these compounds and/or their pharmacological profile are not completely satisfactory. For example, miglustat is capable of crossing the blood brain barrier (BBB), but does not achieve levels in the CNS that exceed its IC₅₀ and many of its effects are either off target or due to its potential activity as a chemical chaperone for beta-glucocerebrosidase. Eliglustat has greater potency than miglustat at inhibiting GCS, but cannot cross the BBB. Accordingly diseases which require a therapeutic drug to cross the BBB cannot be treated. Consequently, there is an ongoing need to provide new compounds that effectively and selectively inhibit GCS, and, in some embodiments, are capable of crossing the BBB. Selected compounds of structural formula (I) exhibit these beneficial properties. At the same time, there remains a need to treat non-CNS diseases such as Gaucher type 1 and Fabry with potent GCS inhibitors that do not cross the BBB efficiently. Selected compounds of structural formula (I) exhibit this property.

To demonstrate the ability of the present GCS inhibitors to reduce glycolipid accumulation in lysosomes and to assess their ability to cross the BBB, compounds of the invention were prepared and studied in in vitro and in vivo assays. The in vitro assays measured the inhibition of glucosylceramide formation in a cellular homogenate and the reduction in the glucosylceramide content of Madin Darby canine kidney (MDCK) cells 24 hours following addition of inhibitor. The in vivo assay measured the reduction in brain and liver glucosylceramide and glucosylsphingosine levels in mice on a D409V background concurrently treated with conduritol B epoxide (CBE), an irreversible inhibitor of GBA (beta-glucocerebrosidase). Compounds of structural formula (I) also are more potent GCS inhibitors in cells and exhibit an improved metabolic stability compared to prior GCS inhibitors.

Compounds of structural formula (I) were found to inhibit GCS in vitro at low nanomolar concentrations and to reduce the levels of glycosphingolipids in vivo with varying degrees of selectivity for brain vs liver.

### Assays

### GCS inhibition in MDCK cell homogenates (Lysate IC50, Table 1)

IC50 values for new compounds of the invention were determined as reported in Shayman J. A., Abe A. 2000. Glucosylceramide synthase: assay and properties. Methods Enzymol.311: 42-49.

### GCS inhibition in MDCKII cells (MDCKII IC50, Table 1)

MDCKII cells were routinely maintained in medium consisting of Opti-MEM/F12 (1:1), 5% FBS, 100U/mL of penicillin, 100 µg/mL streptomycin and 200 mM L-glutamine. MDCKII cells were newly thawed from frozen ampules every two months.

Stock solutions of water-insoluble glycosphingolipid inhibitors (100 mM) were prepared by dissolving each inhibitor into 100% ethanol as previously described (3). The inhibitor-ethanol solutions then were diluted 50X into 2 mM delipidated bovine serum albumin-phosphate buffered saline solution to make water-soluble glycosphingolipid inhibitor-bovine serum albumin complexes. The inhibitor-bovine serum album complexes were sterile-filtered and stored at -20°C. Prior to use, portions of the inhibitor-bovine serum albumin complexes were further diluted with Opti-F12 to make treatment solutions. Equal amounts of bovine serum albumin and ethanol were added into the control cultures. Cells (5 × 10⁵) were seeded into 10-cm culture dishes containing 10 ml of Opti-F12 with 5% FBS. After 24 hours, the medium was replaced with fresh serum-free Opti-F12 medium, and cells were exposed to candidate GCS inhibitors at concentrations of 0, 1, 3, 10, 30, 100 and 300 nM for 24 hours.

### Cell lipid analysis

Following inhibitor treatment, whole cellular lipids of wild type MDCKII cells were extracted as previously described in detail (10). Briefly, cells were washed with ice-cold phosphate buffered saline, fixed by methanol, and collected with rubber scraper. Chloroform was then added to yield a theoretical ratio of chloroform: methanol: water at 1:2:0.8 (v/v/v) to form a mono-phase. Cell debris and proteins were removed by centrifugation at 2200 × g for 30 min. The supernatants were portioned by adding chloroform and 0.9% NaCl. The lower organic phases containing neutral glycosphingolipids lipids were washed with methanol and 0.9% NaCl, and subjected to base- and acid-hydrolysis (10). A portion of purified glycosphingolipids normalized to 100 nmol of total phospholipids was analyzed by high performance thin layer chromatography. The thin layer chromatography separations were processed twice. The plate pretreated with 1% sodium borate was first developed in a solvent system consisting of chloroform/methanol (98/2, v/v). After air drying, the plate was then developed in a solvent system containing chloroform/methanol/water (70/30/4, v/v/v). The levels of glucosylceramide were detected by charring with 8% cupric sulfate in 8% phosphoric acid, and quantified by densitometric scanning using ImageJ, NIH Image. Image data was analyzed, and the IC₅₀ of each inhibitor was calculated using GraphPad Prism (version 5.03). Results are summarized in Table 1.

### Reduction of brain and liver glucosylceramide and glucosylsphingosine in CBE mice

The reduction by inhibitors of GCS in brain glucosylceramide and glucosylsphingosine content was determined by the use of mice with *Gba1* mutant alleles, termed D409V/null. These are knock-in mice with a *Gba1* point mutation encoding Valine (V) 409 for the wild type (WT) Aspartate (D) on one missense allele and a null heteroallele [D409V/null (9V/null)]. The D409V/null and WT littermates were of mixed gender and mixed, but matched, genetic backgrounds of C57BL/129Sv/FVB. (Xu YH, Sun Y, Barnes S, Grabowski GA (2010) Comparative therapeutic effects of velaglucerase alfa and imiglucerase in a Gaucher disease mouse model. PLoS One 5: e10750.) Mice were treated with intraperitoneal conduritol B epoxide (CBE), an irreversible inhibitor of beta-glucocerebrosidase (25mg/kg/day) for all groups beginning at post-partum day 15. Mice were concurrently treated with GCS inhibitor or vehicle control for 14 days. The mice were sacrificed 2 hours following the last injection and tissues including brain, liver, spleen, kidney and lung were analyzed for glucosylceramide and glucosylsphingosine by mass spectrometry.

### Mouse tissue lipid analysis

Lipid extractions of liver, kidney, and brain were performed as previously described (7). Briefly, frozen liver (about 0.5 g), two kidneys (about 0.3 g) and whole brain (about 0.4 g) were individually homogenized in sucrose buffer (250 mM sucrose, pH 7.4, 10 mM HEPES and 1 mM EDTA), at 0.2 g tissue/1 mL of sucrose buffer, with a Tri-R homogenizer. Each 0.8 mL of homogenate was mixed with 2 mL of methanol and 1 mL of chloroform, bath sonicated for 1 min and incubated at room temperature for 1 h. Tissue debris were removed by centrifugation at 2,400 × gravity for 30 min. The pellets were reextracted by mixing with 1 mL of methanol, 0.5 mL of chloroform and 0.4 mL of 0.9% NaCl (chloroform/methanol/0.9% NaCl, 1:2:0.8), incubated at room temperature for 1 h and centrifuged at 2,400 × gravity for another 30 min. Two extracts were combined and mixed with 4.5 mL of chloroform and 1.2 mL of 0.9% NaCl (chloroform/methanol/0.9% NaCl, 2:1:0.8). After centrifugation at 800 × gravity for 5 min, lower layer was washed with 3 mL of methanol and 2.4 mL of 0.9% NaCl. Second washing was carried with 3 mL of methanol, 2 mL of water and 0.4 mL of 0.9% NaCl followed by a 5 min centrifugation at 800 × gravity. The resultant lower phase was collected and dried under a stream of N₂ gas.

The analysis of neutral glycosphingolipids from mouse liver, kidney, and brain was processed after alkaline methanolysis. Kidney lipids were incubated with 2 mL of chloroform and 1 ml of 0.21N NaOH in methanol for 2 h (kidney) or 7.5 h (liver and brain) at RT. The lipid extract was normalized to 0.5 µmol of total phospholipid phosphate (liver and kidney) or 2 µmol of total phospholipid phosphate (brain) for high performance thin layer chromatography analysis. After alkaline methanolysis, the brain lipids were passed through a silica gel column (7). Borate-impregnated thin layer chromatography plates were developed in a two solvent system. Plates were first developed in chloroform/methanol (98:2, v/v). The plates loaded with kidney and liver lipids were then developed in chloroform/methanol/water (64:24:4, v/v/v), and brain lipids were further separated in chloroform/methanol/water (60:30:6, v/v/v). GlcCer levels were quantified by comparison to known standards.

### Assay Results

The activity of compounds of structural formula (I) at inhibiting GlcCer production in vitro is summarized in Table 1.

**Table 1**

| **Example** | **Structure** | **Lysate IC50 (nM)** | **MDCKII IC50 (nM)** |
|---|---|---|---|
| 1 | 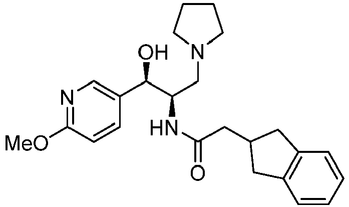 | >1000 | >1000 |
| 2 | 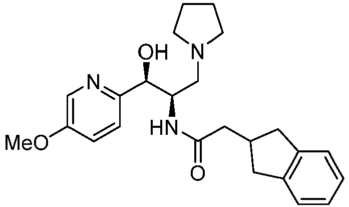 | 24.8 | 6.8 |
| 3 | 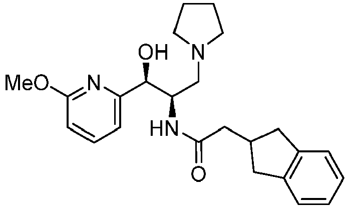 | 198 | 30.5 |
| 4 | 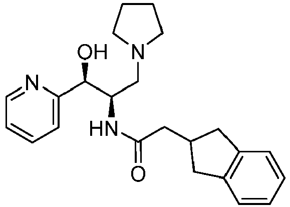 | 152 | 16.9 |
| 5 | 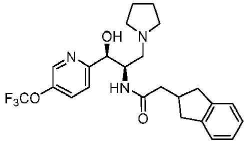 | 249 | 6.5 |
| 6 | 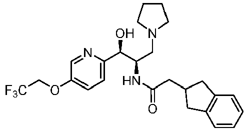 | 21.7 | 2.4 |
| 7 | 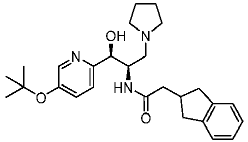 | 16.3 | 7.9 |
| 8 | 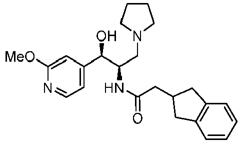 | 56 | 94.6 |
| 9 | 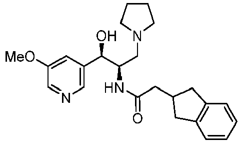 | >5000 | >1000 |
| 10 | 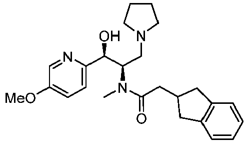 | 37.4 | 15.8 |
| 11 | 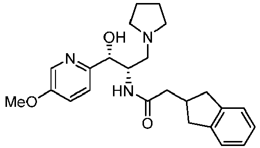 | >2000 | |
| 12 | 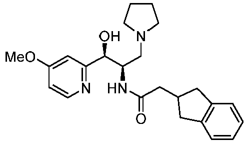 | 24896 | 79.5 |
| 13 | 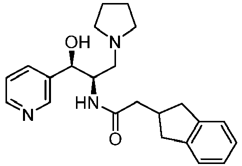 | 182 | 594.0 |
| 14 | 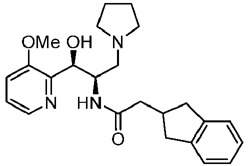 | >2000 | 444.0 |
| 15 | 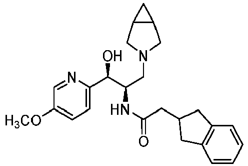 | 21.3 | 9.3 |
| 16 | 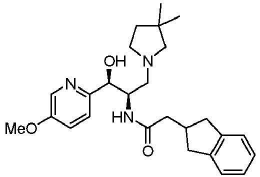 | >2000 | >1000 |
| 17 | 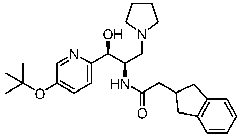 | 11.3 | 4.6 |
| 18 | 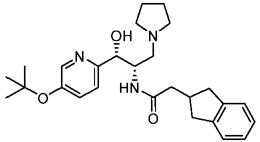 | >2000 | |
| 19 | 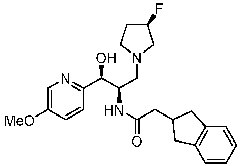 | 82 | 13.9 |
| 20 | 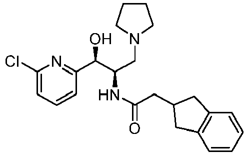 | 131 | 61.0 |
| 21 | 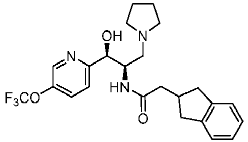 | 77 | 6.5 rac |
| 22 | 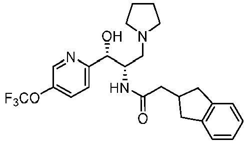 | >1000 | |
| 23 | 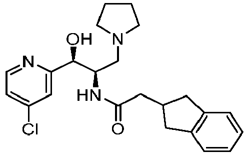 | 16.1 | 3.8 |
| 24 | 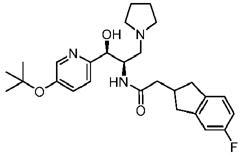 | 568 | 23.8 |
| 25 | 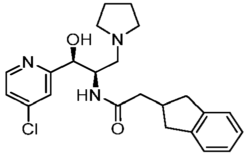 | 36.4 | 0.8 |
| 26 | 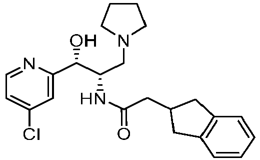 | >2000 | |
| 27 | 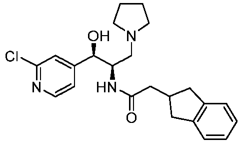 | | 66.6 |

The activity of selected compounds of structural formula (I) at inhibiting GlcCer production in vivo is summarized in Table 2.

**Table 2**

| Example | CBE mouse dose (µmol/kg) | CBE mouse GlcCer reduction in brain (%) | CBE mouse GlcCer reduction in liver (%) |
|---|---|---|---|
| 1 | 109 | 37 | 50 |
| 17 | 100 | 23 | 34 |
| 25 | 72 | 30 | 36 |

The pharmacokinetics of selected compounds of structural formula (I) and structurally-related compounds in the prior art lacking an aromatic nitrogen atom are summarized in Table 3. This shows that incorporation of nitrogen into the aromatic ring can lower the brain/plasma ratio in mice, and indicates that selected compounds of the invention may be advantageous when treating glycolipid storage diseases in the periphery, such as Gaucher disease 1 and Fabry.

**Table 3**

| Example | Structure | Plasma drug AUC^{a} (hr*ng/mL) | Brain drug AUC^{a} (hr*ng/g) | Brain AUC/Plasma AUC |
|---|---|---|---|---|
| | 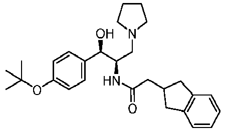 | 1071 | 619 | 0.58 |
| 17 | 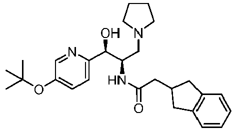 | 902 | 308 | 0.34 |
| | 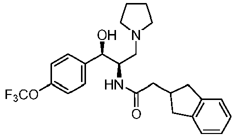 | 1308 | 1046 | 0.80 |
| 21 | 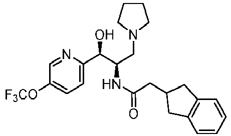 | 1122 | 398 | 0.35 |
| | 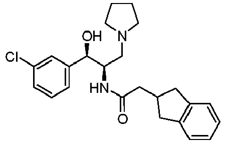 | 1539 | 1109 | 0.72 |
| 25 | 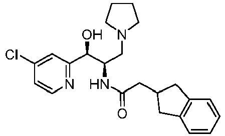 | 1037 | 173 | 0.17 |
| 1 | 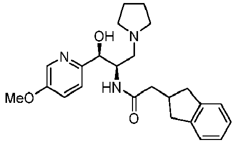 | 895 | 94 | 0.11 |

| | | | | |
|---|---|---|---|---|
| ^{a}Drug levels measured in mice over 7 hrs following single IP injection at 10 mg/kg | | | | |

### USES AND COMPOSITIONS

The present invention provides GCS inhibitors, as exemplified by compounds of structural formula (I), for the treatment of a variety of diseases and conditions wherein inhibition of GCS has a beneficial effect. In one embodiment, the present invention relates to a use in treating an individual suffering from a disease or condition wherein inhibition of the GCS provides a benefit comprising administering a therapeutically effective amount of a compound of structural formula (I) to an individual in need thereof.

The compounds of structural formula (I) therefore can be used to treat a variety of diseases and conditions where inhibition of GCS provides a benefit. Examples of such diseases and condition include, but are not limited to, Tay-Sachs disease, type I, II, and III Gaucher disease, Sandhoff disease, and Fabry's disease; Parkinson's disease (J.R. Mazzulli et al., Cell 146:37-52, July 8, 2011); type 2 diabetes; renal hypertrophy or hyperplasia associated with diabetic nephropathy; elevated plasma TNF-α; elevated blood glucose levels; elevated glycated hemoglobin levels; lupus; and a glomerular disease selected from the group consisting of mesangial proliferative glomerulonephritis, collapsing glomerulopathy, proliferative lupus nephritis, crescentic glomerulonephritis, and membranous nephropathy, or viral illness.

A compound of structural formula (I) also can be used to treat disorders involving cell growth and division, including cancer, collagen vascular diseases, atherosclerosis, and the renal hypertrophy of diabetic individuals (U.S. Patent Nos. 6,916,802 and 5,849,326, each incorporated herein by reference); to inhibit the growth of arterial epithelial cells (U.S. Patent Nos. 6,916,802 and 5,849,326, each incorporated herein by reference); to treat patients suffering from infections (M. Svensson et al., Infect. And Immun., 62:4404-4410 (1994)); to prevent a host, i.e., patient, from generating antibodies against the tumor (J. Inokuchi et al., Cancer Lett., 38:23-30 (1987); and to treat tumors (S. Hakomori Cancer Cells 3:461-470 (1991).); J. Inokuchi et al., Cancer Res., 50L6731-6737 (1990).); and (M. Ziche et al., Lab Invest., 67:711-715 (1992)). A compound of structural formula (I) further can be used to treat a polycystic kidney disease, including both autosomal dominant and recessive forms (T.A. Natoli et al., Nat. Med. 16:788-792 (2010)).

A use of the present invention can be accomplished by administering a compound of structural formula (I) as the neat compound or as a pharmaceutical composition. Administration of a pharmaceutical composition, or neat compound of structural formula (I), can be performed during or after the onset of the disease or condition of interest. Typically, the pharmaceutical compositions are sterile, and contain no toxic, carcinogenic, or mutagenic compounds that would cause an adverse reaction when administered. Further provided are kits comprising a compound of structural formula (I) and, optionally, a second therapeutic agent useful in the treatment of diseases and conditions wherein inhibition of GCS provides a benefit, packaged separately or together, and an insert having instructions for using these active agents.

In many embodiments, a compound of structural formula (I) is administered in conjunction with a second therapeutic agent useful in the treatment of a disease or condition wherein inhibition of GCS provides a benefit. The second therapeutic agent is different from the compound of structural formula (I). A compound of structural formula (I) and the second therapeutic agent can be administered simultaneously or sequentially to achieve the desired effect. In addition, the compound of structural formula (I) and second therapeutic agent can be administered from a single composition or two separate compositions.

The second therapeutic agent is administered in an amount to provide its desired therapeutic effect. The effective dosage range for each second therapeutic agent is known in the art, and the second therapeutic agent is administered to an individual in need thereof within such established ranges.

A compound of structural formula (I) and the second therapeutic agent can be administered together as a single-unit dose or separately as multi-unit doses, wherein the compound of structural formula (I) is administered before the second therapeutic agent or *vice versa.* One or more dose of the compound of structural formula (I) and/or one or more dose of the second therapeutic agent can be administered. The compounds of structural formula (I) therefore can be used in conjunction with one or more second therapeutic agents, for example, but not limited to, enzyme replacement therapy, gene therapy, and isofagamine.

In a use in treating type 2 diabetes, the second therapeutic agent can be one or more of insulin (e.g., NOVOLIN^{®}, NOVOLOG^{®}, VELOSULIN^{®}); a sulfonylurea (e.g., DIABINESE^{®}, GLUCOTROL^{®}, GLUCOTROL XL^{®}, DIABETA^{®}, AMARYL^{®}, ORINASE^{®}, TOLINASE^{®}, MICRONASE^{®}, and GLYNASE^{®}); metformin; an [alpha]-glucosidase inhibitor (e.g., GLYSET^{®}); a thiazolidinedione (e.g., ACTOS^{®} and AVANDIA^{®}); nateglinide (STARLIX^{®}); repaglinide (PRANDIN^{®}), and combination drugs such as AVANDAMET^{®} (AVANDIA^{®} and metformin).

In a use in treating Parkinson's disease, the second therapeutic agent can be one or more of carbidopallevodopa therapy; a dopamine agonist (apomorphine hydrochloride, bromocriptine, rotigotine, pramipexole, ropinirole, pergolide), an anticholinergic (benzotropine mesylate, trihexyphenidyl hydrochloride, procyclidine), an MAO-B inhibitor (selegiline, rasagiline), a COMT inhibitor (entacapone, tulcapone), and other medications including non-prescription, over-the-counter therapeutics (amantadine, rivastigmine tartrate, creatine, coenzyme Q10).

The diseases and conditions that can be treated in accordance to the invention include, for example, Gaucher disease, Fabry disease, Tay-Sachs disease, polycystic kidney disease, and diabetes. In particular, type II and type III Gaucher disease can be treated because compounds of structural formula (I) are capable of crossing the BBB. Prior GCS inhibitors either were incapable of crossing the BBB or had low potency and selectivity, and accordingly various diseases associated with glycolipid accumulation could not be treated.

In the present use, a therapeutically effective amount of one or more compound of structural formula (I), typically formulated in accordance with pharmaceutical practice, is administered to a human being in need thereof. Whether such a treatment is indicated depends on the individual case and is subject to medical assessment (diagnosis) that takes into consideration signs, symptoms, and/or malfunctions that are present, the risks of developing particular signs, symptoms and/or malfunctions, and other factors.

A compound of structural formula (I) can be administered by any suitable route, for example by oral, buccal, inhalation, sublingual, rectal, vaginal, intracisternal or intrathecal through lumbar puncture, transurethral, nasal, percutaneous, i.e., transdermal, or parenteral (including intravenous, intramuscular, subcutaneous, intracoronary, intradermal, intramammary, intraperitoneal, intraarticular, intrathecal, retrobulbar, intrapulmonary injection and/or surgical implantation at a particular site) administration. Parenteral administration can be accomplished using a needle and syringe or using a high pressure technique.

Pharmaceutical compositions include those wherein a compound of structural formula (I) is administered in an effective amount to achieve its intended purpose. The exact formulation, route of administration, and dosage is determined by an individual physician in view of the diagnosed condition or disease. Dosage amount and interval can be adjusted individually to provide levels of a compound of structural formula (I) that is sufficient to maintain therapeutic effects.

Toxicity and therapeutic efficacy of the compounds of structural formula (I) can be determined by standard pharmaceutical procedures in cell cultures or experimental animals, e.g., for determining the maximum tolerated dose (MTD) of a compound, defined as the highest dose that causes no toxicity in animals. The dose ratio between the maximum tolerated dose and therapeutic effects (e.g. inhibiting of tumor growth) is the therapeutic index. The dosage can vary within this range depending upon the dosage form employed, and the route of administration utilized. Determination of a therapeutically effective amount is well within the capability of those skilled in the art, especially in light of the detailed disclosure provided herein.

A therapeutically effective amount of a compound of structural formula (I) required for use in therapy varies with the nature of the condition being treated, the length of time that activity is desired, and the age and the condition of the patient, and ultimately is determined by the attending physician. Dosage amounts and intervals can be adjusted individually to provide plasma levels of a compound of structural formula (I) that are sufficient to maintain the desired therapeutic effects. The desired dose conveniently can be administered in a single dose, or as multiple doses administered at appropriate intervals, for example as one, two, three, four or more subdoses per day. Multiple doses often are desired, or required. For example, a compound of structural formula (I) can be administered at a frequency of: four doses delivered as one dose per day at four-day intervals (q4d × 4); four doses delivered as one dose per day at three-day intervals (q3d × 4); one dose delivered per day at five-day intervals (qd × 5); one dose per week for three weeks (qwk3); five daily doses, with two days rest, and another five daily doses (5/2/5); or, any dose regimen determined to be appropriate for the circumstance.

A compound of structural formula (I) used in the present invention can be administered in an amount of about 0.005 to about 500 milligrams per dose, about 0.05 to about 250 milligrams per dose, or about 0.5 to about 100 milligrams per dose. For example, a compound of structural formula (I) can be administered, per dose, in an amount of about 0.005, 0.05, 0.5, 5, 10, 20, 30, 40, 50, 100, 150, 200, 250, 300, 350, 400, 450, or 500 milligrams, including all doses between 0.005 and 500 milligrams.

The dosage of a composition containing a GCS inhibitor of structural formula (I), or a composition containing the same, can be from about 1 ng/kg to about 200 mg/kg, about 1 µg/kg to about 100 mg/kg, or about 1 mg/kg to about 50 mg/kg. The dosage of a composition can be at any dosage including, but not limited to, about 1 µg/kg. The dosage of a composition may be at any dosage including, but not limited to, about 1 µg/kg, 10 µg/kg, 25 µg/kg, 50 µg/kg, 75 µg/kg, 100 µg/kg, 125 µg/kg, 150 µg/kg, 175 µg/kg, 200 µg/kg, 225 µg/kg, 250 µg/kg, 275 µg/kg, 300 µg/kg, 325 µg/kg, 350 µg/kg, 375 µg/kg, 400 µg/kg, 425 µg/kg, 450 µg/kg, 475 µg/kg, 500 µg/kg, 525 µg/kg, 550 µg/kg, 575 µg/kg, 600 µg/kg, 625 µg/kg, 650 µg/kg, 675 µg/kg, 700 µg/kg, 725 µg/kg, 750 µg/kg, 775 µg/kg, 800 µg/kg, 825 µg/kg, 850 µg/kg, 875 µg/kg, 900 µg/kg, 925 µg/kg, 950 µg/kg, 975 µg/kg, 1 mg/kg, 5 mg/kg, 10 mg/kg, 15 mg/kg, 20 mg/kg, 25 mg/kg, 30 mg/kg, 35 mg/kg, 40 mg/kg, 45 mg/kg, 50 mg/kg, 60 mg/kg, 70 mg/kg, 80 mg/kg, 90 mg/kg, 100 mg/kg, 125 mg/kg, 150 mg/kg, 175 mg/kg, or 200 mg/kg. The above dosages are exemplary of the average case, but there can be individual instances in which higher or lower dosages are merited, and such are within the scope of this invention. In practice, the physician determines the actual dosing regimen that is most suitable for an individual patient, which can vary with the age, weight, and response of the particular patient.

The compounds of the present invention typically are administered in admixture with a pharmaceutical carrier selected with regard to the intended route of administration and standard pharmaceutical practice. Pharmaceutical compositions for use in accordance with the present invention are formulated in a conventional manner using one or more physiologically acceptable carriers comprising excipients and auxiliaries that facilitate processing of compounds of structural formula (I).

These pharmaceutical compositions can be manufactured, for example, by conventional mixing, dissolving, granulating, dragee-making, emulsifying, encapsulating, entrapping, or lyophilizing processes. Proper formulation is dependent upon the route of administration chosen. When a therapeutically effective amount of the compound of structural formula (I) is administered orally, the composition typically is in the form of a tablet, capsule, powder, solution, or elixir. When administered in tablet form, the composition additionally can contain a solid carrier, such as a gelatin or an adjuvant. The tablet, capsule, and powder contain about 0.01% to about 95%, and preferably from about 1% to about 50%, of a compound of structural formula (I). When administered in liquid form, a liquid carrier, such as water, petroleum, or oils of animal or plant origin, can be added. The liquid form of the composition can further contain physiological saline solution, dextrose or other saccharide solutions, or glycols. When administered in liquid form, the composition contains about 0.1% to about 90%, and preferably about 1% to about 50%, by weight, of a compound of structural formula (I).

When a therapeutically effective amount of a compound of structural formula (I) is administered by intravenous, cutaneous, or subcutaneous injection, the composition is in the form of a pyrogen-free, parenterally acceptable aqueous solution. The preparation of such parenterally acceptable solutions, having due regard to pH, isotonicity, stability, and the like, is within the skill in the art. A preferred composition for intravenous, cutaneous, or subcutaneous injection typically contains, an isotonic vehicle.

Compounds of structural formula (I) can be readily combined with pharmaceutically acceptable carriers well-known in the art. Such carriers enable the active agents to be formulated as tablets, pills, dragees, capsules, liquids, gels, syrups, slurries, suspensions and the like, for oral ingestion by a patient to be treated. Pharmaceutical preparations for oral use can be obtained by adding the compound of structural formula (I) to a solid excipient, optionally grinding the resulting mixture, and processing the mixture of granules, after adding suitable auxiliaries, if desired, to obtain tablets or dragee cores. Suitable excipients include, for example, fillers and cellulose preparations. If desired, disintegrating agents can be added.

A compound of structural formula (I) can be formulated for parenteral administration by injection, e.g., by bolus injection or continuous infusion. Formulations for injection can be presented in unit dosage form, e.g., in ampules or in multidose containers, with an added preservative. The compositions can take such forms as suspensions, solutions, or emulsions in oily or aqueous vehicles, and can contain formulatory agents such as suspending, stabilizing, and/or dispersing agents.

Pharmaceutical compositions for parenteral administration include aqueous solutions of the active agent in water-soluble form. Additionally, suspensions of a compound of structural formula (I) can be prepared as appropriate oily injection suspensions. Suitable lipophilic solvents or vehicles include fatty oils or synthetic fatty acid esters. Aqueous injection suspensions can contain substances which increase the viscosity of the suspension. Optionally, the suspension also can contain suitable stabilizers or agents that increase the solubility of the compounds and allow for the preparation of highly concentrated solutions. Alternatively, a present composition can be in powder form for constitution with a suitable vehicle, e.g., sterile pyrogen-free water, before use.

A compound of structural formula (I) also can be formulated in rectal compositions, such as suppositories or retention enemas, e.g., containing conventional suppository bases. In addition to the formulations described previously, the compound of structural formula (I) also can be formulated as a depot preparation. Such long-acting formulations can be administered by implantation (for example, subcutaneously or intramuscularly) or by intramuscular injection. Thus, for example, the compounds of structural formula (I) can be formulated with suitable polymeric or hydrophobic materials (for example, as an emulsion in an acceptable oil) or ion exchange resins.

In particular, the compounds of structural formula (I) can be administered orally, buccally, or sublingually in the form of tablets containing excipients, such as starch or lactose, or in capsules or ovules, either alone or in admixture with excipients, or in the form of elixirs or suspensions containing flavoring or coloring agents. Such liquid preparations can be prepared with pharmaceutically acceptable additives, such as suspending agents. The compounds of structural formula (I) also can be injected parenterally, for example, intravenously, intramuscularly, subcutaneously, or intracoronarily. For parenteral administration, the GCS inhibitors are best used in the form of a sterile aqueous solution which can contain other substances, for example, salts or monosaccharides, such as mannitol or glucose, to make the solution isotonic with blood.

Also disclosed are kits which comprise one or more compounds or compositions packaged in a manner that facilitates their use to practice uses of the invention. In one simple embodiment, the kit includes a compound or composition described herein for the specific therapeutic use(e.g., a composition comprising a compound of structural formula (I) and an optional second therapeutic agent), packaged in a container, such as a sealed bottle or vessel, with a label affixed to the container or included in the kit that describes use of the compound or composition to practice the use of the invention. Preferably, the compound or composition is packaged in a unit dosage form. The kit further can include a device suitable for administering the composition according to the intended route of administration.

Prior GCS inhibitors possessed properties that hindered their development as therapeutic agents. In accordance with an important feature of the present invention, compounds of structural formula (I) were synthesized and evaluated as inhibitors for GCS, and, in particular, for having an ability to cross the BBB. The present GCS inhibitors are characterized by inhibition of GCS at low nanomolar concentrations, high specificity, and the absence of β-glucocerebrosidase binding.

### REFERENCES

1. N. W. Barton et al., N Engl J Med 324, 1464-1470, (1991).
2. N.S. Radin, Glycoconj J 13, 153-157, (1996).
3. J.A. Shayman et al., Methods Enzymol 311, 373-387, (2000).
4. N.J. Weinreb et al., Am J Hematol 80, 223-229, (2005).
5. J.A. Shayman, Drugs of the Future 35, 613-621, (2010).
6. E. Lukina et al., Blood 116(20):4095-8, (2010).
7. A. Abe et al., J Clin Invest 105, 1563-1571, (2000).
8. Y. Liu et al., The Journal of clinical investigation 103, 497-505, (1999).
9. J.A. Shayman et al., Methods Enzymol 311, 42-49, (2000).
10. L. Shu et al., J Biol Chem 278, 31419-31425, (2003).

## Claims

1. A compound having a structure 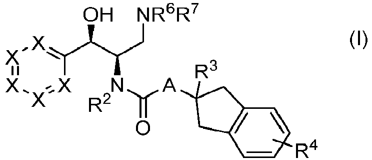
wherein each X independently is CH, cR1, or N, and with the proviso that one and only one X must be N;
A is CH₂ or O;
R¹ is H, halogen, or OR⁵;
R² is H, C₁-C₅ alkyl, or C₃-C₆ cycloalkyl;
R³ is H or CH₃;
R⁴ is H or F;
R⁵ is C₁-C₄ alkyl or C₃-C₆ cycloalkyl, optionally substituted with one or more F; and
R⁶ and R⁷ independently, are H or C₁-C₃ alkyl or R⁶ and R⁷ are taken together with the nitrogen atom to which they are attached to form a C₄-C₈ heterocycloalkyl or heterobicycloalkyl ring, optionally substituted with F or CH_{3;}
or a pharmaceutically acceptable salt thereof.

2. The compound of claim 1 wherein 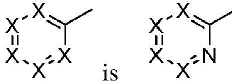 and X is CH or CR¹.

3. The compound of claim 1 wherein R¹ is -H, -OC(CH₃)₃, -OCH₃, -OCF₃, -OCH₂CF₃, or -Cl.

4. The compound of claim 1 wherein R² is H or CH₃.

5. The compound of claim 1 wherein NR⁶R⁷ is

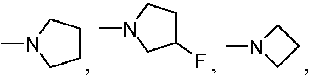

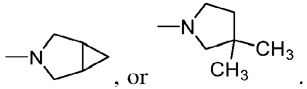

6. The compound of claim 1 wherein

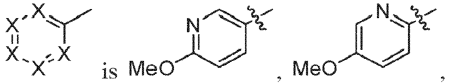

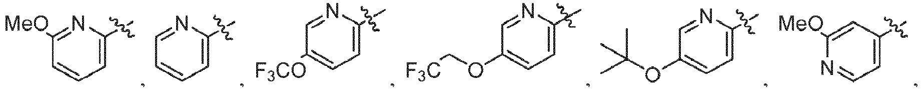

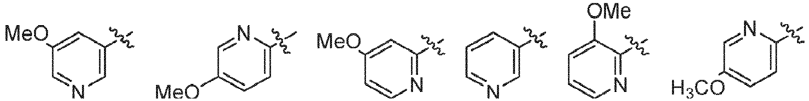

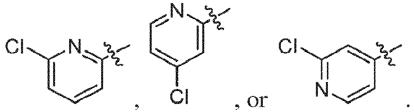

7. A compound of claim 1 selected from the group consisting of 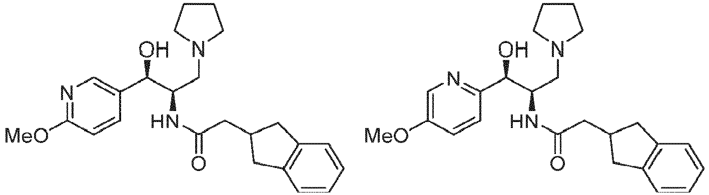 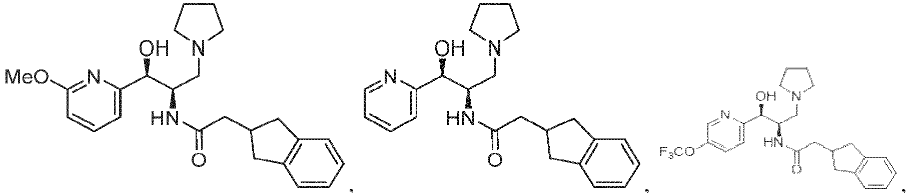 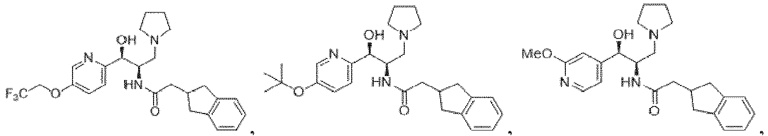 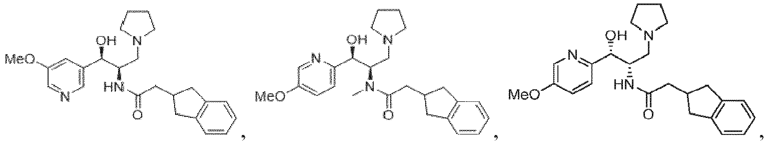 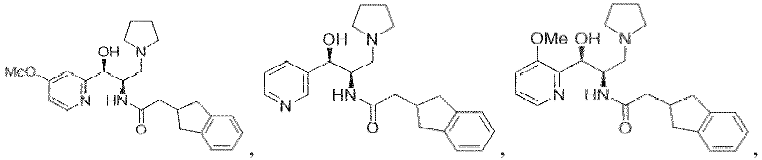 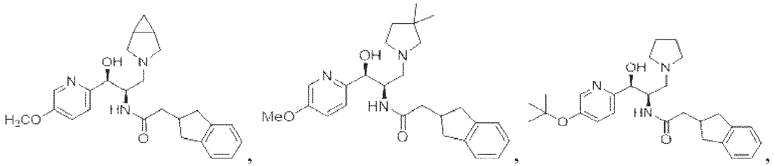 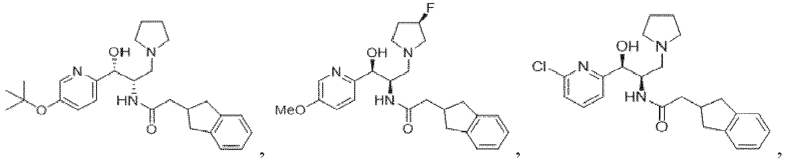 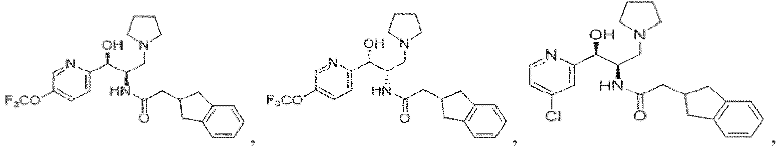 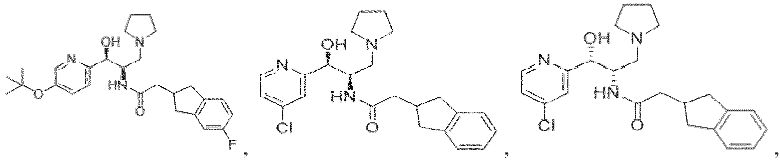 and

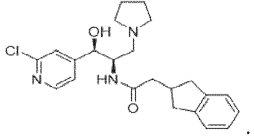

8. A pharmaceutical composition comprising a compound of any of claims 1-7 and a pharmaceutically acceptable carrier or vehicle.

9. A composition comprising (a) compound of any of claims 1-7, (b) a second therapeutic agent useful in the treatment of a Gaucher disease, Fabry disease, type II diabetes, Sandhoff disease, Tay-Sachs disease, or Parkinson's disease, and (c) an optional excipient and/or pharmaceutically acceptable carrier.

10. A compound according to any of claims 1 to 7 or a composition according to claim 8 or 9 for use in treating a disease or condition wherein inhibition of glucosylceramide synthase (GCS) provides a benefit.

11. The compound or composition for use according to claim 10, wherein a second therapeutic agent useful in the treatment of the disease or condition is administered, wherein the second therapeutic agent is preferably one or more of enzyme replacement therapy, gene therapy, and isotagamine.

12. The compound or composition for use according to claim 10 or 11 wherein the disease or condition is a Gaucher disease, Fabry disease, Sandhoff disease, Tay-Sachs disease, Parkinson's disease, multiple myeloma, ADPCD, type 2 diabetes, hypertrophy or hyperplasia associated with diabetic neuropathy, an elevated plasma TNF-α level, an elevated blood glucose level, an elevated glycated hemoglobin level, a glomerular disease, or lupus.

13. The compound or composition for use according to claim 12, wherein the Gaucher disease is type I, type II, or type III Gaucher disease.

14. The compound or composition for use according to claim 12 wherein the glomerular disease is selected from the group consisting of mesangial proliferative glomerulonephritis, collapsing glomerulopathy, proliferative lupus nephritis, crescentic glomerulonephritis, and membranous nephropathy.

15. The compound or composition for use according to claim 10 or 11 wherein the disease or condition is a disorder involving cell growth, a disorder involving cell division, a collagen vascular disease, atherosclerosis, renal hypertrophy in a diabetic individual, a growth of arterial epithelial cells, an infection, a tumor, and a polycystic kidney disease.

16. The compound or composition for use according to claim 15 wherein the disease or condition is a cancer or an autosomal dominant or recessive form of the polycystic kidney disease.

17. A compound according to any of claims 1 to 7 or a composition according to claim 8 or 9 for use in inhibiting glucosylceramide synthase or lowering a glycosphinolipid concentration in an individual in need thereof.

## Patentansprüche

1. Eine Verbindung mit einer Struktur 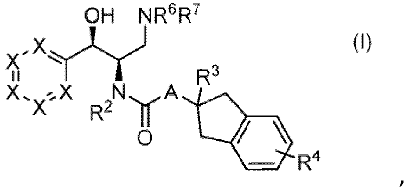
wobei jedes X unabhängig für CH, CR¹ oder N steht und mit der Maßgabe, dass ein und nur ein X für N stehen muss;
A für CH₂ oder O steht;
R¹ für H, Halogen oder OR⁵ steht;
R² für H, C₁-C₅-Alkyl oder C₃-C₆-Cycloalkyl steht;
R³ für H oder CH₃ steht;
R⁴ für H oder F steht;
R^{S} für C₁-C₄-Alkyl oder C₃-C₆-Cycloalkyl steht, welches gegebenenfalls mit einem oder mehreren F substituiert ist; und
R⁶ und R⁷ unabhängig für H oder C₁-C₃-Alkyl stehen oder R⁶ und R⁷ mit dem Stickstoffatom, an welches sie gebunden sind, zusammengenommen werden, um einen C₄-C₈-Hetercycloalkyl- oder Heterobicycloalkylring zu bilden, welcher gegebenenfalls mit F oder CH₃ substituiert ist;
oder ein pharmazeutisch verträgliches Salz davon.

2. Die Verbindung gemäß Anspruch 1, wobei 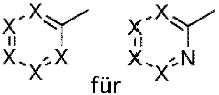 steht und X für CH oder CR¹ steht.

3. Die Verbindung gemäß Anspruch 1, wobei R¹ für -H, -OC(CH₃)₃, -OCH₃, -OCF₃, -OCH₂CF₃ oder -Cl steht.

4. Die Verbindung gemäß Anspruch 1, wobei R² für H oder CH₃ steht.

5. Die Verbindung gemäß Anspruch 1, wobei NR⁶R⁷ für

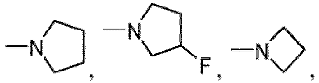

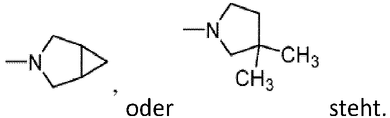

6. Die Verbindung gemäß Anspruch 1, wobei 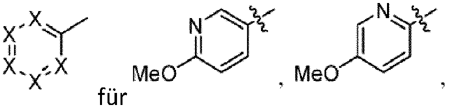 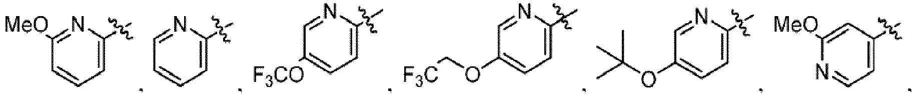 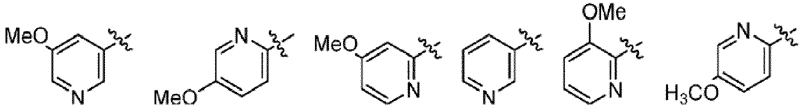 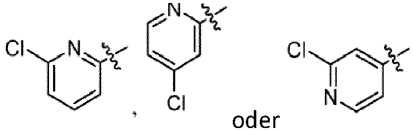 steht.

7. Eine Verbindung gemäß Anspruch 1, ausgewählt aus der Gruppe bestehend aus 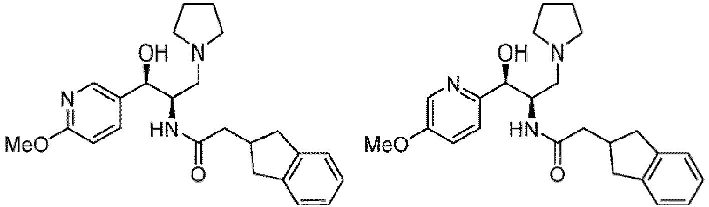 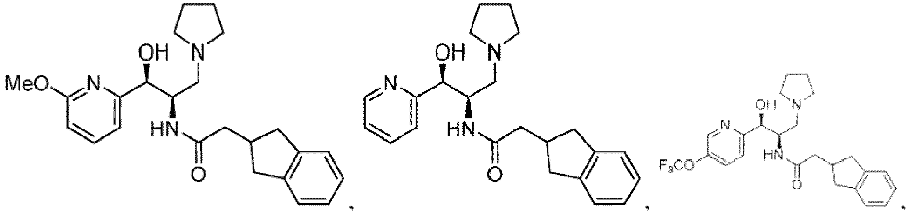 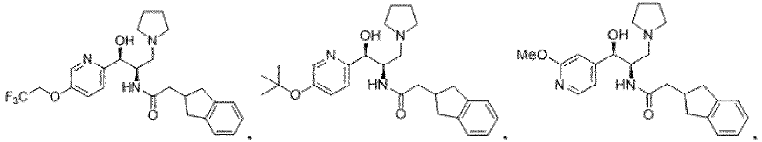 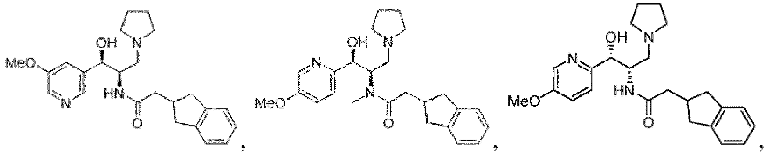 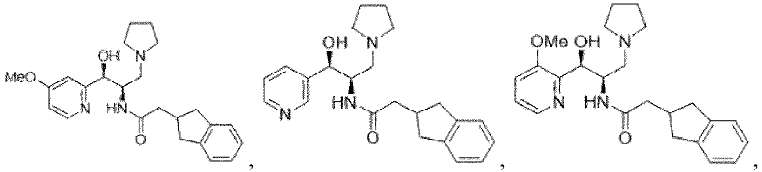 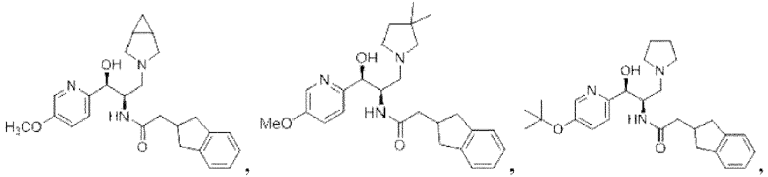 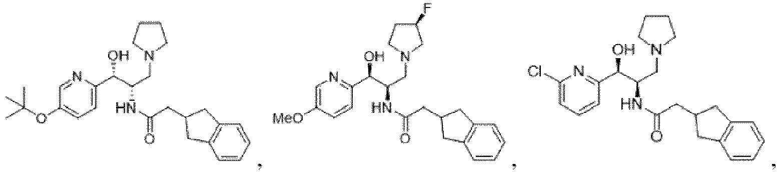 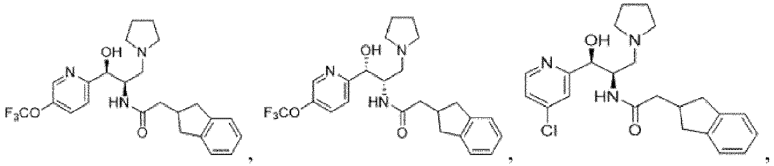 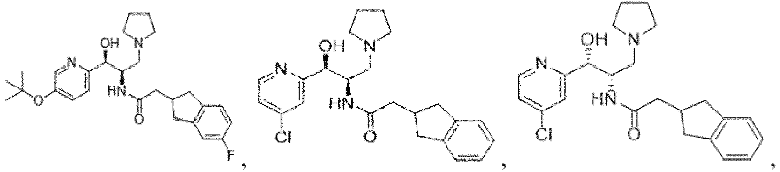 und

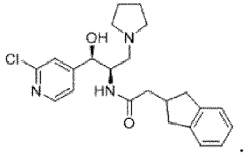

8. Ein Arzneimittel, umfassend eine Verbindung gemäß einem der Ansprüche 1 bis 7 und einen pharmazeutisch verträglichen Träger oder Trägerstoff.

9. Eine Zusammensetzung, umfassend (a) eine Verbindung gemäß einem der Ansprüche 1 bis 7, (b) einen zweiten therapeutischen Wirkstoff, welcher bei der Behandlung von Morbus Gaucher, Morbus Fabry, Diabetes Typ II, Sandhoff-Krankheit, Tay-Sachs-Syndrom oder Parkinson-Krankheit verwendbar ist, und (c) einen optionalen Exzipienten und/oder einen pharmazeutisch verträglichen Träger.

10. Eine Verbindung gemäß einem der Ansprüche 1 bis 7 oder eine Zusammensetzung gemäß Anspruch 8 oder 9 zur Verwendung bei der Behandlung einer Erkrankung oder eines Zustandes, bei welcher/welchem die Hemmung von Glucosylceramid-Synthase (GCS) einen Vorteil bietet.

11. Die Verbindung oder die Zusammensetzung zur Verwendung gemäß Anspruch 10, wobei ein zweites therapeutisches Mittel, welches bei der Behandlung der Erkrankung oder des Zustandes verwendbar ist, verabreicht wird, wobei das zweite therapeutische Mittel vorzugsweise eine oder mehrere von Enzymersatztherapie, Gentherapie und Isotagamin ist.

12. Die Verbindung oder Zusammensetzung zur Verwendung gemäß Anspruch 10 oder 11, wobei die Erkrankung oder der Zustand Morbus Gaucher, Morbus Fabry, Sandhoff-Krankheit, Tay-Sachs-Syndrom, Parkinson-Krankheit, multiples Myelom, ADPCD, Diabetes Typ II, Hypertrophie oder Hyperplasie, welche mit diabetischer Neuropathie in Zusammenhang steht, ein erhöhter Plasma-TNF-α-Spiegel, ein erhöhter Blutzuckerspiegel, ein erhöhter glykosylierter Hämoglobinspiegel, eine glomeruläre Erkrankung oder Lupus ist.

13. Die Verbindung oder Zusammensetzung zur Verwendung gemäß Anspruch 12, wobei es sich bei Morbus Gaucher um Morbus Gaucher vom Typ I, Typ II oder Typ III handelt.

14. Die Verbindung oder Zusammensetzung zur Verwendung gemäß Anspruch 12, wobei die glomeruläre Erkrankung ausgewählt ist aus der Gruppe bestehend aus mesangialer proliferativer Glomerulonephritis, "collapsing" Glomerulopathie, proliferativer Lupusnephritis, halbmondförmiger Glomerulonephritis und membranöser Nephropathie.

15. Die Verbindung oder Zusammensetzung zur Verwendung gemäß Anspruch 10 oder 11, wobei die Erkrankung oder der Zustand eine Störung des Zellwachstums, eine Störung der Zellteilung, eine Kollagengefäßerkrankung, Atherosklerose, Nierenhypertrophie bei einer Person mit Diabetes, Wachstum von arteriellen Epithelzellen, eine Infektion, ein Tumor und eine polyzystische Nierenerkrankung ist.

16. Die Verbindung oder Zusammensetzung zur Verwendung gemäß Anspruch 15, wobei die Erkrankung oder der Zustand Krebs oder eine autosomal-dominante oder -rezessive Form der polyzystischen Nierenerkrankung ist.

17. Eine Verbindung gemäß einem der Ansprüche 1 bis 7 oder eine Zusammensetzung gemäß Anspruch 8 oder 9 zur Verwendung bei der Hemmung von Glucosylceramid-Synthase oder Senkung einer Glycosphinolipid-Konzentration bei einem Patienten, der dieser bedarf.

## Revendications

1. Composé ayant la structure 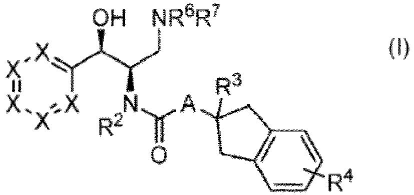
dans lequel chaque X est indépendamment CH, CR¹ ou N, et à condition qu'un et uniquement un X soit N ;
A est CH₂ ou O ;
R¹ est H, un atome d'halogène ou OR⁵ ;
R² est H, un groupe alkyle en C₁ à C₅ ou un groupe cycloalkyle en C₃ à C₆ ;
R³ est H ou CH₃ ;
R⁴ est H ou F ;
R⁵ est un groupe alkyle en C₁ à C₄ ou un groupe cycloalkyle en C₃ à C₆, éventuellement substitué par un ou plusieurs F ; et
R⁶ et R⁷ sont indépendamment H ou un groupe alkyle en C₁ à C₃ ou R⁶ et R⁷ sont pris conjointement avec l'atome d'azote auquel ils sont liés pour former un cycle hétérobicycloalkyle ou hétérocycloalkyle en C₄ à C₈, éventuellement substitué par F ou CH₃ ;
ou un sel pharmaceutiquement acceptable de celui-ci.

2. Composé selon la revendication 1 dans lequel 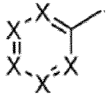 est 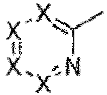 et X est CH ou CR¹.

3. Composé selon la revendication 1 dans lequel R¹ est -H, - OC(CH₃)₃, -OCH₃, -OCF₃, -OCH₂CF₃ ou -CI.

4. Composé selon la revendication 1 dans lequel R² est H ou CH₃.

5. Composé selon la revendication 1 dans lequel NR⁶R⁷ est

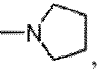

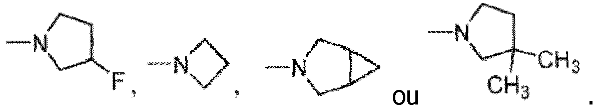

6. Composé selon la revendication 1 dans lequel 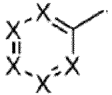 est

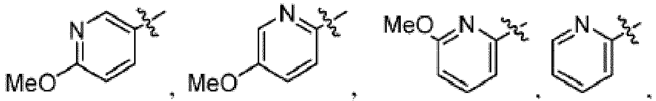

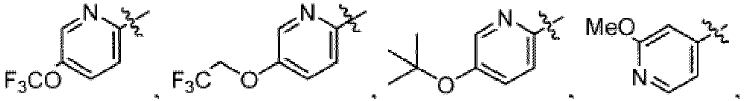

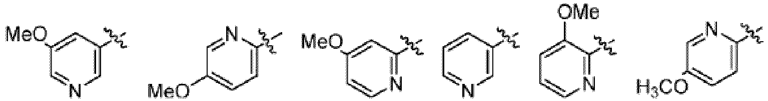

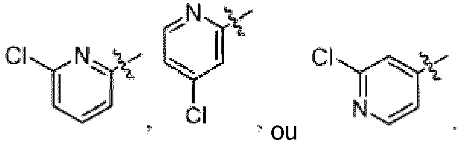

7. Composé selon la revendication 1 choisi dans le groupe constitué par 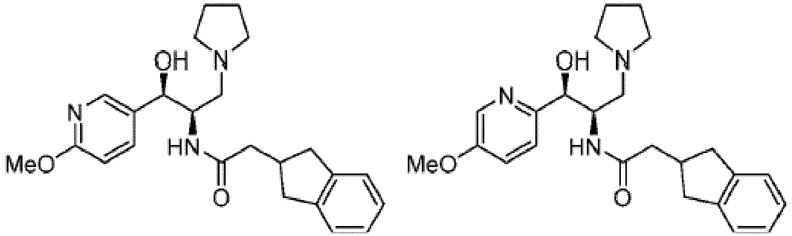 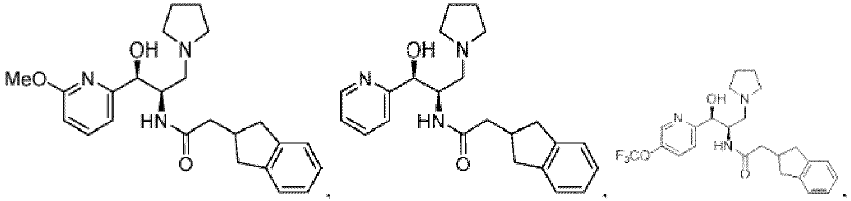 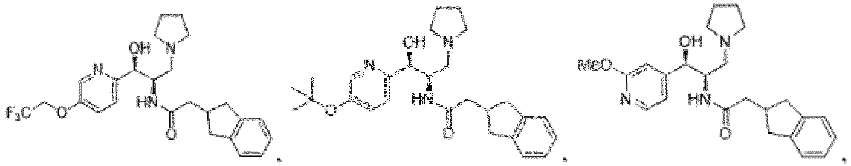 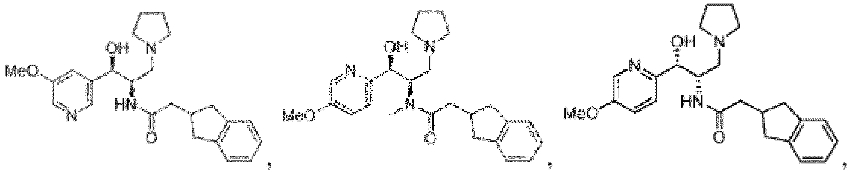 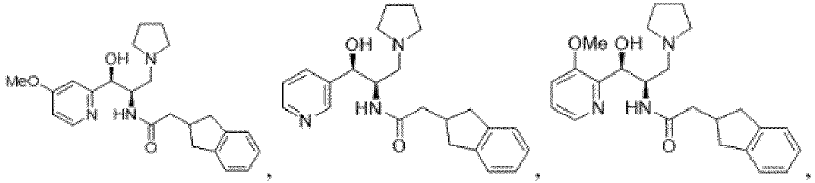 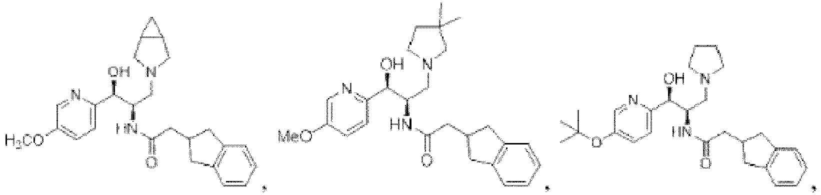 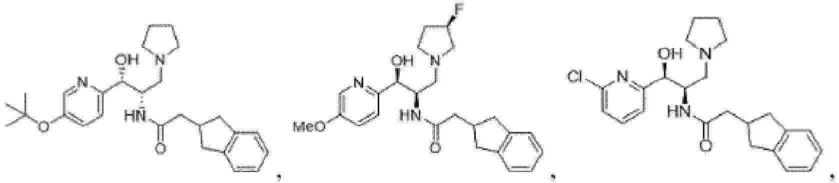 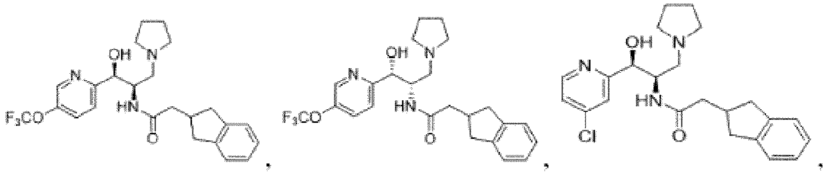 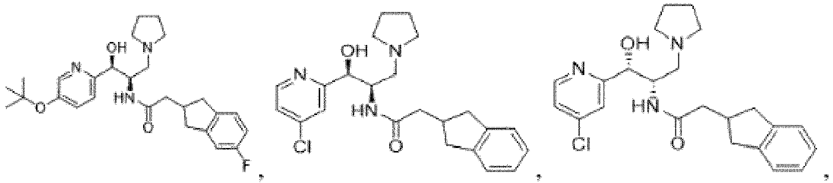 et

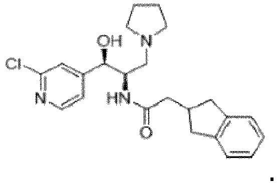

8. Composition pharmaceutique comprenant un composé selon l'une quelconque des revendications 1 à 7 et un support ou un véhicule pharmaceutiquement acceptable.

9. Composition comprenant (a) un composé selon l'une quelconque des revendications 1 à 7, (b) un second agent thérapeutique utile dans le traitement d'une maladie de Gaucher, de la maladie de Fabry, du diabète de type II, de la maladie de Sandhoff, de la maladie de Tay-Sachs ou de la maladie de Parkinson, et (c) un excipient et/ou un support pharmaceutiquement acceptable facultatifs.

10. Composé selon l'une quelconque des revendications 1 à 7 ou composition selon la revendication 8 ou 9 pour une utilisation dans le traitement d'une maladie ou d'une affection dans laquelle l'inhibition de glucosylcéramide synthase (GCS) offre un bénéfice.

11. Composé ou composition pour une utilisation selon la revendication 10, dans lesquels un second agent thérapeutique utile dans le traitement de la maladie ou affection est administré, dans lequel le second agent thérapeutique est de préférence un ou plusieurs parmi la thérapie enzymatique substitutive, la thérapie génique et l'isotagamine.

12. Composé ou composition pour une utilisation selon la revendication 10 ou 11, dans lesquels la maladie ou l'affection est une maladie de Gaucher, la maladie de Fabry, la maladie de Sandhoff, la maladie de Tay-Sachs, la maladie de Parkinson, le myélome multiple, la PKRAD, le diabète de type 2, l'hypertrophie ou l'hyperplasie associée à la neuropathie diabétique, un taux plasmatique élevé de TNF-α, une glycémie élevée, un taux d'hémoglobine glyquée élevé, une maladie glomérulaire ou le lupus.

13. Composé ou composition pour une utilisation selon la revendication 12, dans lesquels la maladie de Gaucher est la maladie de Gaucher de type I, de type II ou de type III.

14. Composé ou composition pour une utilisation selon la revendication 12, dans lesquels la maladie glomérulaire est choisie dans le groupe constitué par la glomérulonéphrite proliférative mésangiale, la glomérulopathie avec collapsus, la néphrite lupique proliférative, la glomérulonéphrite en croissant et la néphropathie membraneuse.

15. Composé ou composition pour une utilisation selon la revendication 10 ou 11, dans lesquels la maladie ou l'affection est un trouble impliquant la croissance cellulaire, un trouble impliquant la division cellulaire, une maladie vasculaire du collagène, l'athérosclérose, l'hypertrophie rénale chez un individu diabétique, une croissance de cellules épithéliales artérielles, une infection, une tumeur et une maladie polykystique des reins.

16. Composé ou composition pour une utilisation selon la revendication 15, dans lesquels la maladie ou l'affection est un cancer ou une forme autosomique dominante ou récessive de la maladie polykystique des reins.

17. Composé selon l'une quelconque des revendications 1 à 7 ou composition selon la revendication 8 ou 9 pour une utilisation dans l'inhibition de la glucosylcéramide synthase ou la diminution d'une concentration en glycosphinolipides chez un individu en ayant besoin.
